(19) Europäisches Patentamt
European Patent Office
Office européen des brevets

(11) Numéro de publication: **0 169 787 B1**

(12) **FASCICULE DE BREVET EUROPEEN**

(45) Date de publication du fascicule du brevet: **06.09.89**

(51) Int. Cl.⁴: **C 07 H 21/00**, A 61 K 31/70

(21) Numéro de dépôt: **85401506.2**

(22) Date de dépôt: **22.07.85**

(54) **Application d'oligonucléotides liés à un agent intercalant à titre de médicament.**

(30) Priorité: **25.07.84 FR 8411795**

(43) Date de publication de la demande: **29.01.86 Bulletin 86/5**

(45) Mention de la délivrance du brevet: **06.09.89 Bulletin 89/36**

(84) Etats contractants désignés: **AT BE CH DE FR GB IT LI LU NL SE**

(56) Documents cités:
**EP-A- 0 117 777**

**PROCEEDINGS OF THE NATIONAL ACADEMY OF SCIENCES, vol. 81, no. 11, juin 1984, pages 3297-3301;US; U. ASSELINE et al.: "Nucleic acid-binding molecules with high affinity and base sequence specificity: Intercalating agents covalently linked to oligodeoxynucleotides."**
**BIOCHEMISTRY, vol. 17, no. 14, 11 juillet 1978, pages 2915-2919; The American Chemical Society,US; J. REUBEN et al.: "Structure of mutagen nucleic acid complexes in solution. Proton chemical complexes in solution. Proton chemical shifts in 9-aminoacridine complexes with dG-dC, dC-dG, and dA-dT-dG-dC-dA-dT"**
**CHEMICAL ABSTRACTS, vol. 101, no. 1, 2 juillet 1984,**

(73) Titulaire: **CENTRE NATIONAL DE LA RECHERCHE SCIENTIFIQUE (CNRS), 15, Quai Anatole France, F-75007 Paris (FR)**

(72) Inventeur: **Helene, Claude, 252 Rue Haute, F-45590 Saint Cyr en Val (FR)**
Inventeur: **Nguyen, Thanh Thuong, 31 Route de Tigy Vienne en Val, F-45510 Tigy (FR)**

(74) Mandataire: **Warcoin, Jacques et al, Cabinet Régimbeau 26, avenue Kléber, F-75116 Paris (FR)**

(56) Documents cités: (suite)
**page 3486, réf. no. 3474x, Columbus, Ohio,US; U. ASSELINE et al.: "Oligodeoxynucleotides covalently linked to intercalating dyes as base sequence-specific ligands. Influence of dye attachment site."**

**Le dossier contient des informations techniques présentées postérieurement au dépôt de la demande et ne figurant pas dans le présent fascicule.**

EP 0 169 787 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne l'application d'oligonucléotides liés à un agent intercalant au blocage sélectif de l'expression d'un gène.

Plus particulièrement, la présente invention concerne à titre de médicament des composés oligonucléotides liés à un agent intercalant pour le blocage sélectif d'une séquence d'acide nucléique, dans laquelle ces composés sont constitués par un oligonucléotide ou un oligodésoxynucléotide constitué d'un enchaînement de nucléotides naturel ou modifié complémentaire de la séquence d'acide nucléique à bloquer sélectivement, sur lequel se trouve fixé par une liaison covalente un groupe d'intercalation.

Il convient de remarquer que dans le cadre de la présente demande, les termes «blocage sélectif» ont un sens très général mais s'appliquent, de préférence, à un «blocage in vivo». Ainsi, il peut s'agir du blocage d'une séquence impliquée dans l'initiation, la propagation ou la terminaison de la réplication d'un acide nucléique, de la transcription d'un ou plusieurs gènes et/ou de leur traduction.

Mais, il peut s'agir aussi d'une action de protection qui rend la séquence «bloquée» inaccessible pour une enzyme ou un composé chimique par exemple.

Parmi les composés utilisables dans la présente invention, il faut citer plus particulièrement les composés décrits dans la demande, publiée ultérieurement à la date dont bénéficie la présente demande, EP 117 777 de formule:

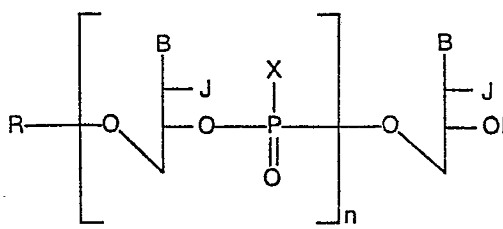

(I)

dans laquelle:

les radicaux B peuvent être identiques ou différents et représentent chacun une base d'un acide nucléique naturelle ou modifiée;

les radicaux X, qui peuvent être identiques ou différents, représentent chacun un oxoanion $O^{\ominus}$, un thioanion $S^{\ominus}$, un groupe alkyle, un groupe alcoxy, aryloxy, alcoxy ou alkyle substitué par un hétérocycle azoté, un groupe aminoalkyle, un groupe aminoalcoxy, un groupe thioalkyle ou un groupement –Y–Z;

R et R', qui peuvent être identiques ou différents, représentent chacun un atome d'hydrogène ou un groupement –Y–Z dans lequel:

Y représente un radical alcoylène droit ou ramifié –alk– ou un radical

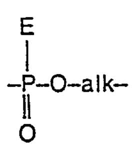

dans lequel E peut avoir les mêmes significations que X; ou bien un radical –Y''–O–Y' où Y'' et Y' peuvent avoir les significations données pour Y; et

Z est un radical correspondant à un agent d'intercalation;

J représente un atome d'hydrogène ou un groupe hydroxy;

n est un nombre entier y compris O.

Il convient de remarquer que la formule I représente un enchaînement de nucléotides qui peuvent être identiques ou différents, n indiquant simplement le nombre de nucléotides compris dans la molécule; n est de préférence un nombre compris entre 1 et 50 et de préférence entre 1 et 25.

Les agents d'intercalation Z sont des composés connus dans les techniques touchant aux acides nucléiques, il s'agit de composés capables de «s'intercaler» dans la structure des ADN ou des ARN.

Ces agents d'intercalation sont, en général, constitués par des composés polycycliques ayant une configuration plane tels que l'acridine, la furocoumarine, la daunomycine, la 1,10-phénanthroline, la phénanthridinium, les porphyrines, l'ellipticine ou l'ellipticinium et leurs dérivés.

Parmi les significations de Z, trois seront utilisées plus particulièrement:

– le groupe oxy-8-psoralène (Z'),

– le groupe méthoxy-2-chloro-6-amino-9-acridine (Z''), et

– le groupe amino-9-acridine (Z''').

Le radical B est, de préférence, constitué par une base naturelle d'un acide nucléique, par exemple la thymine, l'adénine, la cytosine, la guanine ou l'uracile; mais, il est également possible d'utiliser des bases d'acides nucléiques modifiées, comme la 2-amino-adénine et ses dérivés substitués, par exemple sur le $N^6$ par un groupe aminoalkylène ou par un groupe azidophénylalkylène; ou la guanine substituée sur le $O^6$, par exemple par un groupe (ω-alkylène)-9-acridine; ou la 8-(ω-aminoalkyl)-aminoadénine et ses dérivés substitués sur le $NH_2$ en ω par un groupe acridine; ou des dérivés halogénés ou azidés, par exemple la 5-bromo-uacile ou la 8-azidoadénine. En outre, il est possible d'utiliser des dérivés photoactivables des bases.

Le radical X, bien qu'il représente de préférence un oxoanion, peut avoir d'autres significations; lorsque le radical X représente un groupement alkyle, il s'agit de préférence d'un groupement alkyle inférieur en $C_1$ à $C_7$ et, par exemple, les groupements éthyle, méthyle ou propyle; lorsque le radical X représente un groupe alcoxy, il s'agit de préférence d'un groupement alcoxy inférieur en $C_1$ à $C_7$, par exemple le groupement méthoxy ou éthoxy; lorsque X représente un groupement amino-alkyle ou amino-alcoxy, il peut s'agir d'un groupement amino-alkyle mono-substitué, disubstitué ou bien d'un radical amino sous forme de sel d'ammonium quaternaire, dans ces conditions, les substituants sont, de préférence, des radicaux alkyles inférieurs comme définis précédemment; quant à la chaîne alkyle ou alcoxy reliant le radical amino au phosphore, il s'agit de

préférence d'une chaîne droite ou ramifiée comportant de 1 à 10 atomes de carbone; lorsque le radical alkyle ou alcoxy est substitué par un hétérocycle azoté, il s'agit notamment de cycle saturé à 5–6 chaînons comportant un atome d'azote qui peut être quaternaire; enfin, lorsque le radical X est un radical thioalkyle, il s'agit de préférence d'un radical thioalkyle inférieur, c'est-à-dire qui comporte entre 1 et 7 atomes de carbone.

R et R′, outre la signification hydrogène, peuvent représenter notamment:

a) –alk–Z

b) 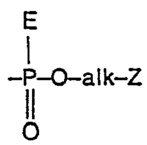

c) 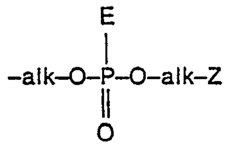

d) 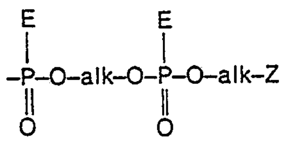

Le radical –alk– est de préférence un radical alcoylène droit ou ramifié ayant de 1 à 10 atomes de carbone.

La présente invention concerne également l'application des composés précédents sous forme de sel avec des bases ou des acides, et les composés sous forme racémique, ou sous forme d'isomères R ou S optiques purifiés ou en mélange.

Les documents EP 117 777, Proc. Natl. Acad. Sci. USA, Vol. 81, (1984) pages 3297–3301 et Chem. Abs. 101: 3474 (EMBO J., (1984) Vol. 3, (4), pages 795–800) ont proposé l'utilisation des composés ci-dessus à titre de sonde ou de moyen de purification d'ADN ou d'ARN ou encore de moyen de diagnostic.

L'objet de la présente invention est, en particulier, l'application de ces composés pour réaliser:

1°) Le blocage spécifique des gènes cellulaires préalablement choisis,

2°) Le blocage sélectif de la réplication et/ou du développement de virus, bactéries ou parasites.

Ce blocage est obtenu par la formation de complexes d'interaction spécifique entre les composés de formule I et les séquences nucléiques complémentaires.

Selon la présente invention, on peut contrôler l'expression d'un gène en utilisant un composé de formule I dont la séquence est complémentaire de celle d'une région spécifique du gène ou de l'ARN messager correspondant. La réplication d'un ADN ou d'un ARN peut être bloquée par un composé de formule I dont la séquence est complémentaire d'une région de régulation, d'initiation ou de propagation de la réplication.

Si le produit de l'expression du gène visé est vital pour le virus ou pour la bactérie ou pour le parasite, ou si la réplication de l'ADN ou de l'ARN est bloquée, alors les composés de formule A agiront comme substances antivirales ou comme antibiotiques ou comme produits antiparasitaires.

Si le produit de l'expression du gène visé n'est pas vital pour l'organisme, on peut alors moduler ou supprimer sélectivement ses effets. Dans ce cas, les composés de formule I agiront par exemple soit comme substances antitumorales, lorsque le gène visé est un gène oncogène ou un gène impliqué dans l'immortalisation ou la transformation cellulaire, soit comme une substance qui permet de supprimer le caractère de résistance aux antibiotiques, aux antiviraux et aux antiparasitaires lorsque le gène visé est le ou les gènes de résistance correspondants.

Ainsi, on peut, par exemple, bloquer la synthèse de la protéine 32 du bactériophage T4 en utilisant les composés suivants (voir l'exemple XVII):

T–T–T–A–A–$(CH_2)_5$ Z″

T–T–T–A–A–T–T–T–A–A–$(CH_2)_5$ Z″

T–T–T–A–A–T–T–T–A–A–T–T–T–A–A–$(CH_2)_5$ Z″

dont les séquences sont complémentaires de la région située en amont du codon d'initiation sur l'ARN messager (voir tableau III).

De même, l'exemple XVIII montre qu'on peut réprimer le gène de la β-lactamase qui est responsable de la résistance des bactéries aux antibiotiques comportant le cylce β-lactame en utilisant les séquences suivantes:

5′ C–C–C–T–G–A–$(CH_2)_5$ Z″

5′ T–A–A–C–C–C–T–G–A–$(CH_2)_5$ Z″

qui sont complémentaires de la région d'initiation de la transcription de ce gène.

Selon la présente invention, on peut inhiber le développement d'un virus oncogène ou non oncogène en employant un composé de formule I dont la séquence est complémentaire d'une région spécifique d'ADN ou d'ARN viral.

L'heptanucléotide 5′ A–A–A–G–C–A–G–$(CH_2)_5$ Z″ dont la séquence est complémentaire de celle des extrémités 3′ terminales des 8 ARN du virus de la grippe de type A (Desselberger U., Racaniello V.R., Zagra J.J. et Palese P. (1980) Gene 8, 315–328) inhibe totalement l'effet cytopathogène de ce virus (voir exemple XX).

Parmi les trois virus suivants: virus SV 40, virus de la grippe A/WSN et virus de l'herpès, qui ont été soumis à l'action des composés:

5′ T–T–T–T–T–T–T–T–$(CH_2)_6$ Z″,

5′ T–T–T–T–T–T–T–T–Et, et

$HO(CH_2)_6$ Z″,

seul le virus SV 40 qui possède la séquence:

5′ T–T–T–T–T–T–T–T
. . . . . . . .
3′ A–A–A–A–A–A–A–A

au voisinage de l'origine de réplication et de transcription des gènes précoces (Soeda E. et Miura K.I. (1979) FEBS Letters 101, 359–362) est sensible au composé $(T–)_8(CH_2)_6$ Z″.

L'octathymidate $(T–)_8$Et dépourvu du groupe intercalant Z″ est inactif vis-à-vis de ces virus; tandis que l'effet cytotoxique du composé $HO(CH_2)_6$ Z″ est trop important pour que son activité antivirale puisse être mise en évidence (voir exemple XIX).

Les composés de la présente invention possèdent donc à la fois une grande sélectivité et une forte affinité pour les séquences nucléiques cibles. Cette forte affinité spécifique se traduit ainsi par la très faible toxicité des composés de l'invention vis-à-vis des cellules saines (voir exemples XIX et XX).

Ces résultats montrent que les composés de la présente invention peuvent être utilisés soit pour réguler l'expression d'un gène, soit pour bloquer le développement d'un virus, d'une bactérie ou d'un parasite. Les composés de formule I, par leur grande affinité spécifique pour les séquences nucléiques complémentaires, sont à la fois supérieurs aux oligonucléotides seuls qui possèdent une affinité plus faible pour les séquences complémentaires et à l'agent intercalant seul qui est toxique vis-à-vis des cellules saines par manque de spécificité.

Les composés selon la présente invention sont donc applicables à titre de médicament dans le traitement des maladies d'origine virale, bactérienne ou parasitaire, et également à titre d'agent antitumoral. Ils peuvent également être utilisés pour rendre sensibles à différents antibiotiques, antiviraux ou antiparasitaires, des souches jusque – là résistantes en bloquant les gènes responsables de cette résistance.

Les dosages et les voies d'administration de ces composés pourront varier suivant la nature de l'affection traitée et du type d'activité à attendre pour le composé.

La présente invention concerne également des compositions pharmaceutiques comportant à titre de principe actif au moins un médicament tel que décrit précédemment, seul ou en association avec un antibiotique, un antiparasitaire, un antiviral ou un agent antitumoral, ainsi qu'un support acceptable dans le domaine pharmaceutique.

Selon la présente invention, on peut améliorer l'efficacité des composés de formule I par des modifications diverses, par exemple au niveau des groupes phosphoesters, au niveau du bras reliant l'oligonucléotide à l'intercalant, au niveau des bases nucléiques. Par ces modifications, on peut par exemple augmenter l'affinité de ces substances pour les séquences d'acides nucléiques complémentaires, rendre ces composés stables vis-à-vis des nucléases et faciliter leur passage à travers les membranes cellulaires.

On peut augmenter l'affinité des composés de l'invention pour les séquences nucléiques complémentaires en remplaçant le groupe adénine par le groupe amino-2-adénine qui permet de créer une liaison hydrogène supplémentaire avec le 0–2 de la thymine ou de l'uracile des acides nucléiques.

On peut aussi augmenter l'affinité des composés de l'invention pour les séquences nucléiques complémentaires en remplaçant partiellement ou totalement les groupes phosphodiesters soit par des groupes phosphotriesters ou phosphonates neutres, soit par des groupes porteurs de charges positives. Dans le premier cas, cette modification permet de réduire ou de supprimer les répulsions électrostatiques entre les charges négatives des groupes phosphodiesters d'oligonucléotides et les charges négatives de la séquence complémentaire d'acide nucléique. Dans le second cas, les modifications augmentent l'affinité vis-à-vis des séquences complémentaires par attraction électrostatique.

La substitution de n groupes phosphodiesters par n groupes phosphotriesters ou phosphonates conduit à la formation de $n^2$ stéréoisomères dont les propriétés physiochimiques et biochimiques peuvent être très différentes.

Le tableau suivant compare les températures de demi-dissociation des complexes formés entre le poly(rA) et les oligothymidylates I dont le groupe phosphodiester est remplacé ou non par un groupe méthylphosphonate. Ces résultats montrent que la substitution des groupes phosphodiesters des composés I permet d'augmenter leur affinité pour la séquence nucléique complémentaire.

| | Configuration du groupe méthylphosphonate | $T_{1/2}$ (°C) |
|---|---|---|
| 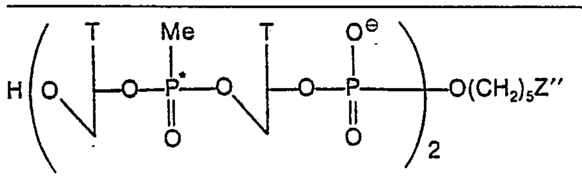 | S<br>R<br>(R,S) | 41<br>9<br>28 |
| 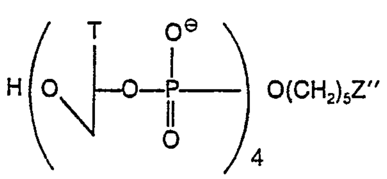 | | 31 |
| 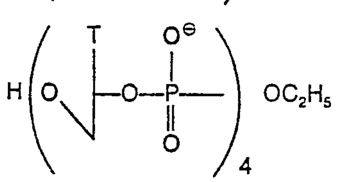 | | ~0 |

Les valeurs $T_{1/2}$ ont été déterminées pour un rapport 1:1 (T:A) à la concentration de $5 \times 10^{-5}$ M en intercalant et dans un tampon à pH 7 contenant $10^{-2}$ M cacodylate de sodium et 0,1 M NaCl.

Selon la présente invention, la substitution des groupes phosphodiesters par des groupes phosphotriesters ou phosphonates neutres, ou mieux, chargés positivement, apporte à la chaîne nucléotidique une plus grande stabilité vis-à-vis des nucléases. Par exemple, les composés répondant à la structure suivante:

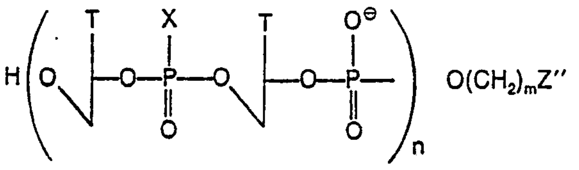

avec X = Me ou OEt

ne sont pas dégradés par les exonucléases telles que l'exonucléase extraite de la rate de veau et du venin de serpent (l'inactivité de cette dernière est due au blocage de l'hydroxyle-3').

Les composés suivants:

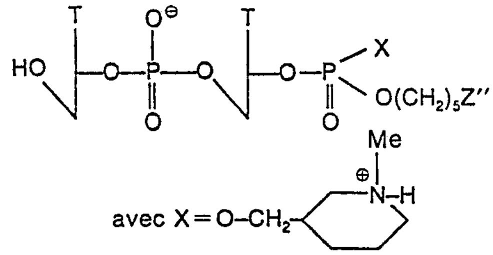

ou $O(CH_2)_3\ \overset{\oplus}{N}Me_3$

sont à la fois stables vis-à-vis de ces exonucléases et de l'endonucléase $P_1$ extraite de Penicillium citrium dans les conditions où le dinucléoside TpT est totalement dégradé par ces enzymes.

La synthèse des nouveaux composés de la présente invention peut être réalisée selon des procédés analogues à ceux déjà décrits dans le brevet français no 8 301 223 du 27 janvier 1983.

Parmi ces composés, on peut citer, par exemple, les composés de formule:

$(T-)_n(CH_2)_nZ$

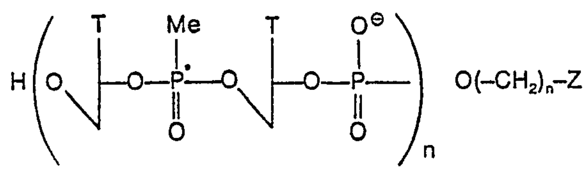

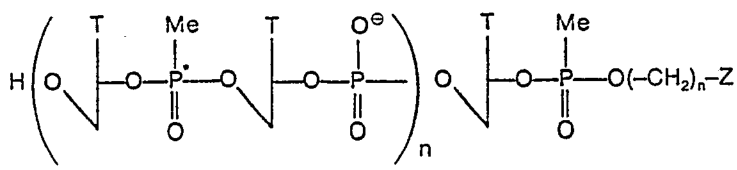

$d[T-G-A-(CH_2)_nZ]$

$d[C-C-C-T-G-A-(CH_2)_nZ]$

$d[T-A-A-C-C-C-T-G-A-(CH_2)_nZ]$

$d[A-A-A-G-C-A-G-(CH_2)_nZ]$

$d[A-G-C-G-A-A-A-G-C-A-G-(CH_2)_nZ]$

$d[A-G-C-A-A-A-A-G-C-A-G-(CH_2)_nZ]$

$d[C-T-G-C-T-T-T-(CH_2)_nZ]$

$d[C-C-T-G-C-T-T-T-T-G-C-(CH_2)_nZ]$

$d[C-C-T-G-C-T-T-T-T-G-C-(CH_2)_nZ]$

$d[C-C-T-G-C-T-T-T-C-G-C(CH_2)_nZ]$

$d[T-C-G-T-C-G-(CH_2)_nZ]$

$d[G-G-C-A-T-C-G-T-C-G-(CH_2)_nZ]$

Les exemples suivants sont destinés à illustrer d'autres caractéristiques et avantages de l'invention, mais ne la limitent évidemment nullement.

EXEMPLE I

T–T–T–T–$(CH_2)_5$ Z'''

1) (DMTr) T∓T∓T∓T∓ $(CH_2)_5$ Z'''

On fait réagir pendant 1 heure à la température ambiante un mélange de 1 équivalent de (DMTr) T∓T∓T∓T–Ar (préparé selon le brevet français no 8 301 223), 1,5 équivalent de (ω-hydroxypentyl-amino)-9 acridine et 2 équivalents de mésithylène-sulfonyltétrazolide (MST) dans la pyridine anhydre. Après avoir détruit l'excès du réactif de couplage par addition de l'eau glacée, le produit est extrait avec du chloroforme puis purifié sur gel de silice en utilisant les mélanges $CH_2Cl_2$, MeOH (99:1 à 90:10, v/v).

2) On fait réagir sous agitation pendant une nuit et à la température ambiante le tétranucléotide préparé précédemment avec une solution molaire en acide benzohydroxamique et en 1,8-diazabicyclo (5,4,0) undec-7-ène (DBU) dans la pyridine anhydre et en utilisant 10 équivalents d'acide benzohydroxamique-DBU par équivalent de phosphoester arylé à déprotéger. On neutralise le milieu réactionnel avec du DOWEX 50 (forme pyridinium), on filtre et lave la résine avec un mélange eau, MeOH (1:1, v/v), puis on chasse le solvant sous vide. Le résidu obtenu est traité avec de l'acide acétique à 80% pendant 1 à 2 heures à la température ambiante. On élimine l'acide acétique sous vide par coévaporation plusieurs fois avec de l'éthanol; on reprend le résidu avec de l'eau puis on lave la phase aqueuse avec de l'éther. Le produit est purifié par chromatographie liquide à haute performance (HPLC) d'abord par échange d'ion, puis en phase inverse. Les temps de rétention du produit purifié sont donnés dans les tableaux I et II.

EXEMPLE II

Octathymidylate dérivé d'acide méthylphosphonique dont la configuration est sous forme de mélange de stéréoisomères.

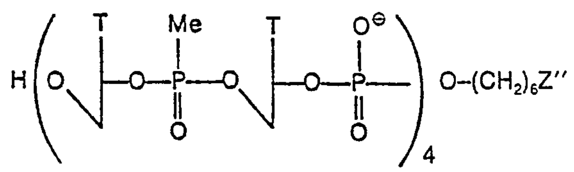

1) 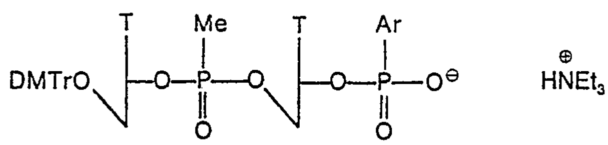

0,2 mmole de dinucléotide 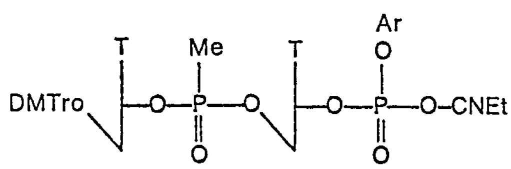

(préparé selon le brevet français no 8 301 223) est traité pendant 2 heures à la température ambiante avec 4 ml d'une solution de pyridine-triéthylamine (2:1, v/v). On chasse le solvant sous vide puis on lave le solide obtenu avec de l'éther.

2) 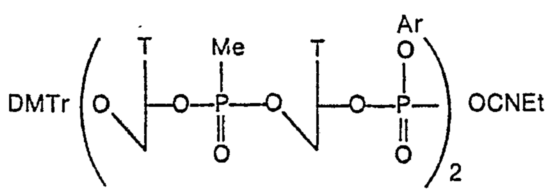

Le dinucléotide

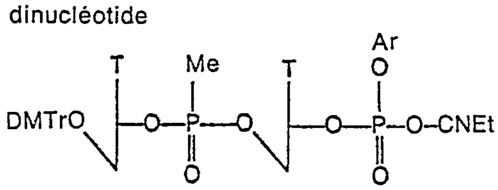

(0,1 mole)

est traité avec 1,5 ml d'acide benzène sulfonique (2% en solution dans le chloroforme, méthanol, 7:3, v/v) à 0 °C pendant 15 minutes. Le mélange est repris avec 20 ml de chloroforme et lavé avec une solution aqueuse de $NaHOC_3$ à 5% puis séché sur $Na_2SO_4$ et concentré sous vide. A ce résidu on ajoute 0,12 mmole de diester préparé d'après II-1. Aprés avoir séché le mélange par coévaporation avec la pyridine, on ajoute au résidu 1,5 ml de pyridine anhydre et 0,3 mmole de MST et on laisse le mélange réactionnel à la température ambiante pendant 2 heures sous agitation puis on termine la préparation comme dans l'exemple I-1. Rendement = 65%.

3) 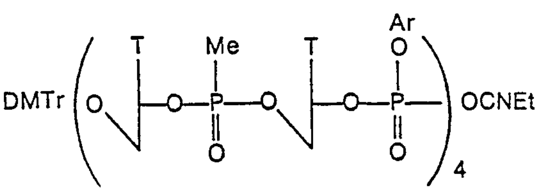

On opère comme dans l'exemple II-1 et l'exemple II-2 en remplaçant le composé

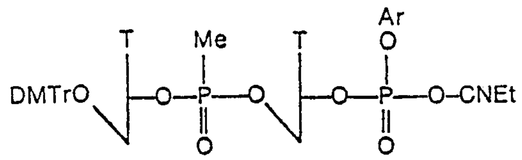

par le composé

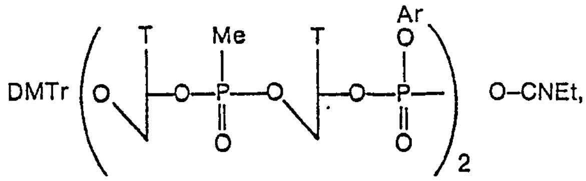

on obtient l'octathymidylate qui est purifié sur silice en présence du système de solvants: $CH_2Cl_2$, eau, acétone (43:2:55, v/v).

4) 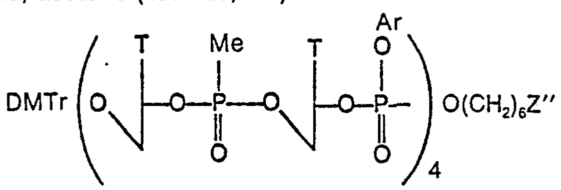

1 équivalent de diester

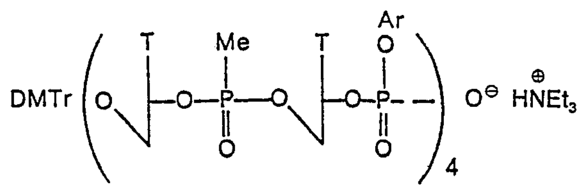

(préparé par action de la triéthylamine sur le triester II-3 selon l'exemple II-1) est couplé avec la méthoxy-2-chloro-6(ω-hydroxyhexylamino)-9 acridine (2 équivalents) en présence de MST (3 équivalents) dans la pyridine anhydre. Après avoir détruit l'excès du réactif de couplage par addition d'eau glacée, le produit est extrait avec du chloroforme puis purifié sur gel de silice en présence du système de solvants: $CH_2Cl_2$, eau, acétone (20:5:75, v/v).

5) La déprotection du composé II-4 est réalisée comme dans l'exemple I-2 sauf pour la désarylation qui nécessite environ 24 heures; le produit est ensuite purifié par chromatographie liquide à haute performance (HPLC). Les tableaux I et II donnent les temps de rétention du produit purifié.

EXEMPLES III et IV

Octathymidylates dérivés d'acide méthylphosphonique dont la configuration est sous forme d'isomère S (III) et d'isomère R (IV)

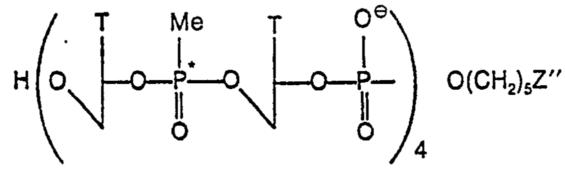

En partant de chacun des isomères S (ouα) et R (ouβ) du dinucléotide

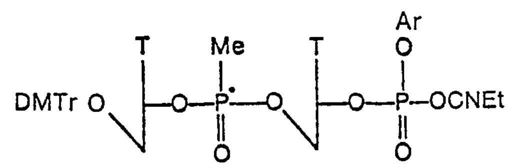

(préparés selon le brevet français no 8 301 223) et en opérant selon l'exemple II en remplaçant la méthoxy-2 chloro-6 (ω-hydroxyhexylamino)-9 acridine par la méthoxy-2 chloro-6 (ω-hydroxypentylamino)-9 acridine, on a préparé les octathymidylates dot les groupes méthylphosphonates possèdent soit la configuration S (III) soit la configuration R (IV).

Les temps de rétention des produits purifiés sont réunis dans les tableaux I et II.

EXEMPLE V

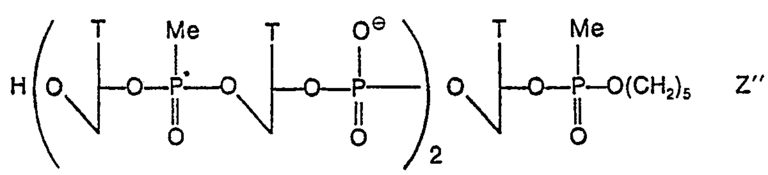

Les groupes méthylphosphonates internucléotidiques possèdent le configuration S.

(1) 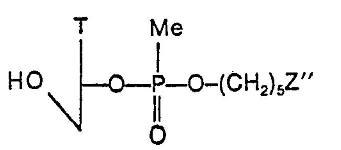

Le 5'-O(diméthoxytrityl)thymidine-3' méthylphosphonate (préparé selon le brevet français no 8 301 223) (1 équivalent) est traité pendant 2 heures sous agitation à la température ambiante avec le méthoxy-2 chloro-6 (ω-hydroxypentylamino)-9 acridine (2 équivalents) en présence de MST (3 équivalents). On détruit l'excès de MST par addition d'eau glacée, extrait le produit formé avec du chloroforme puis on purifie par chromatographie sur gel de silice. Le produit est ensuite détritylé par un traitement avec l'acide benzène sulfonique (2% en solution dans le chloroforme, méthanol (7:3, v/v) à 0 °C pendant 15 minutes. Le mélange est repris avec du chloroforme et lavé avec une solution aqueuse de $NaHCO_3$ à 5% puis séché sur $Na_2SO_4$ et concentré sous vide. Le produit est purifié par chromatographie sur gel de silice.

2) En couplant l'isomère S du tétranucléotide

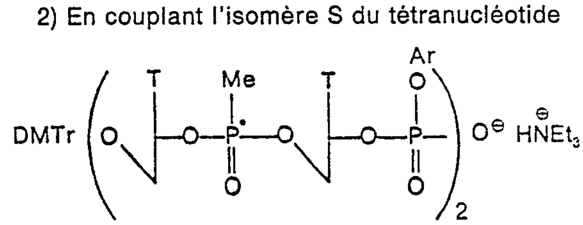

(1 équivalent) avec le composé

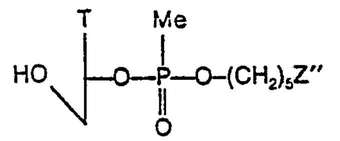

(1,2 équivalent) en présence de MST (3 équivalents) et en opérant comme dans l'exemple I, on a obtenu le pentathymidylate dont le temps de rétention en HPLC est donné par le tableau II.

EXEMPLE VI

d T–G–A–$(CH_2)_5$ Z″

1) d bz A∓ $(CH_2)_5$ Z″

Le composé (DMTr) dbzA∓$(CH_2)_5$Z″ (1 mmole) (brevet français no 8 301 223) est traité pendant 15 minutes à 0°C avec 10 ml d'une solution d'acide benzène sulfonique à 2% dans le chloroforme, méthanol (7:3, v/v). La solution est reprise avec du chloroforme et lavée avec une solution aqueuse de $NaHCO_3$ à 5% puis séchée sur $Na_2SO_4$ et concentrée sous vide.

2) (DMTr) dT∓ibG–Ar

Le dinucléotide (DMTr)dT∓ibG∓CNEt (1,2 mmole) (préparé selon le brevet français no 8 301 223) est traité pendant 2 heures à la température ambiante avec 3 ml d'une solution de pyridine-triéthylamine (2:1, v/v). On chasse le solvant sous vide puis on lave le solide obtenu avec de l'éther.

3) (DMTr) dT∓ibG∓bzA∓$(CH_2)_5$ Z″

Les composés dbzA∓$(CH_2)_5$Z″ et (DMTr)dT∓ibG–Ar sont mélangés puis séchés par coévaporation plusieurs fois avec la pyridine anhydre. A ce résidu on ajoute 10 ml de pyridine et 3 mmoles de MST puis on laisse 1 à 2 heures sous agitation à la température ambiante. On détruit l'excès du réactif de couplage par addition de l'eau glacée, on extrait le produit avec du chloroforme puis on sèche la solution chloroformique sur $Na_2SO_4$. Après avoir chassé le solvant sous vide, le produit est purifié sur gel de silice en utilisant successivement les systèmes de solvant $CH_2Cl_2$,$H_2O$, acétone (46:2:52 et 40:2:58, v/v).

4) Le trinucléotide VI-3 (1 équivalent) est traité pendant une nuit à la température ambiante avec une solution molaire de

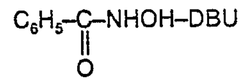

dans la pyridine (on utilise 10 équivalents de

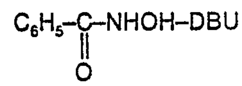

par équivalent de groupe arylphosphoester à déprotéger). A cette solution on ajoute ensuite 2 volumes de solution de soude molaire dans MeOH,$H_2O$ (2:1, v/v) puis on continue l'agitation pendant 2 jours à la température ambiante. Après avoir neutralisé le mélange réactionnel avec une résine anionique (forme pyridinium), on chasse le solvant sous vide, puis on traite le résidu obtenu pendant 1 à 2 heures à la température ambiante avec de l'acide acétique à 80%. On termine la préparation comme dans l'exemple I.

Le temps de rétention du produit purifié est donné dans le tableau II.

EXEMPLES VII à XVI

En utilisant des oligonucléotides intermédiaires préparés selon les procédés décrits dans le brevet français no 8 301 223 et en opérant comme dans l'exemple VI pour les étapes de détritylation, de décyanoéthylation et de couplage, on prépare les oligonucléotides protégés selon les schémas donnés ci-après: on élimine ensuite les groupements protecteurs comme dans l'exemple VI en augmentant la durée de déprotection pour des séquences plus longues (2 jours pour la désarylation avec le couple acide benzohydroxamique-DBU). Les oligonucléotides déprotégés VII à XVI sont ensuite purifiés par échange d'ion puis en phase inverse sur $C_{18}$. Les tableaux I et II donnent les temps de rétention des composés préparés.

EXEMPLE VII

(DMTr)danC–Ar danC∓CNEt

MST

(DMTr)danC∓anC∓CNEt

$H^{\oplus}$

DMTrdanC-Ar danC∓anC∓CNEt

MST

(DMTr)danC∓anC∓anC∓CNEt

$NET_3$

(DMTr)dT∓ibG∓bzA∓$(CH_2)_5$Z″

$H^{\oplus}$

↓ $NET_3$ ↓ $H^{\oplus}$

(DMTr)danC∓anC∓anC–Ar dT∓ibG∓bzA∓$(CH_2)_5Z''$

↓ MST

(DMTr)danC∓anC∓anC∓T∓ibG∓bzA∓$(CH_2)_5Z''$

1) $C_6H_5CNHOH$,DBU (C=O)

2) $OH^{\ominus}$

3) $H^{\oplus}$

d[C–C–C–T–G–A–$(CH_2)_5Z''$]

EXEMPLE VIII

(DMTr)T–Ar dbzA∓bzA∓CNEt

↓ MST

(DMTr)dT∓bzA∓bzA∓CNEt (DMTr)danC∓anC∓anC∓T∓ibG∓bzA∓$(CH_2)_5Z''$

↓ $NEt_3$ ↓ $H^{\oplus}$

(DMTr)dT∓bzA∓bzA–Ar danC∓anC∓anC∓T∓ibG∓bzA∓$(CH_2)_5Z''$

↓ MST

(DMTr)dT∓bzA∓bzA∓anC∓anC∓anC∓T∓ibG∓bzA∓$(CH_2)_5Z''$

1) $C_6H_5CNHOH$,DBU (C=O)

2) $OH^{\ominus}$

3) $H^{\oplus}$

d[T–A–A–C–C–C–T–G–A–$(CH_2)_5Z''$]

EXEMPLE IX

(DMTr)dbzA∓ibG–Ar danC∓bzA∓CNEt

↓ MST

(DMTr)dbzA∓ibG∓anC∓bzA∓CNEt

↓ $H^{\oplus}$

$H^{\oplus}$

(DMTr)dbzA∓bzA–Ar dbzA∓ibG∓anC∓bzA∓CNEt

MST

(DMTr)dbzA∓bzA∓bzA∓ibG∓anC∓bzA∓CNEt

$NEt_3$

(DMTr)dbzA∓bzA∓bzA∓ibG∓anC∓bzA–Ar dibG∓$(CH_2)_5Z''$

MST

(DMTr)dbzA∓bzA∓bzA∓ibG∓anC∓bzA∓ibG∓$(CH_2)_5Z''$

1) $C_6H_5C(=O)NHOH$,DBU

2) $OH^{\ominus}$

3) $H^{\oplus}$

d[A–A–A–G–C–A–G–$(CH_2)_5Z''$]

EXEMPLE X

(DMTr)dbz∓ibG–Ar danC∓ibG∓CNEt

MST

(DMTr)dbzA∓ibG∓anC∓ibG∓CNEt

$NEt_3$

(DMTr)dbzA∓ibG∓anC∓ibG–Ar dbzA∓bzA∓bzA∓ibG∓anC∓bzA∓ibG∓$(CH_2)_5Z''$

MST

(DMTr)dbzA∓ibG∓anC∓ibG∓bzA∓bzA∓bzA∓ibG∓anC∓bzA∓ibG∓$(CH_2)_5Z''$

1) $C_6H_5C(=O)NHOH$,DBU

2) $OH^{\ominus}$

3) $H^{\oplus}$

d[A–G–C–G–A–A–A–G–C–A–G–$(CH_2)_5Z''$]

EXEMPLE XI

(DMTr)dbzA $\mp$ ibG–Ar    danC $\mp$ bzA $\mp$ CNEt

MST

(DMTr)dbzA $\mp$ ibG $\mp$ anC $\mp$ bzA $\mp$ CNEt

$NEt_3$

(DMTr)dbzA $\mp$ ibG $\mp$ anC $\mp$ bzA–Ar    dbzA $\mp$ bzA $\mp$ bzA $\mp$ ibG $\mp$ anC $\mp$ bzA $\mp$ ibG $\mp$ $(CH_2)_5Z''$

MST

(DMTr)dbzA $\mp$ ibG $\mp$ anC $\mp$ bzA $\mp$ bzA $\mp$ bzA $\mp$ bzA $\mp$ ibG $\mp$ anC $\mp$ bzA $\mp$ ibG $\mp$ $(CH_2)_5Z''$

1) $C_6H_5C(=O)NHOH$, DBU

2) $OH^{\ominus}$

3) $H^{\oplus}$

d[A–G–C–A–A–A–A–G–C–A–G–$(CH_2)_5Z''$]

EXEMPLE XII

(DMTr)dibG $\mp$ anC–Ar    T $\mp$ T $\mp$ CNEt

MST

(DMTr)dibG $\mp$ anC $\mp$ T $\mp$ T $\mp$ CNEt

$H^{\oplus}$

(DMTr)danC $\mp$ T–Ar    dibG $\mp$ anC $\mp$ T $\mp$ T $\mp$ CNEt

MST

(DMTr)danC $\mp$ T $\mp$ ibG $\mp$ anC $\mp$ T $\mp$ T $\mp$ CNEt

$NEt_3$

(DMTr)danC $\mp$ T + ibG $\mp$ anC $\mp$ T $\mp$ T–Ar    T $\mp$ $(CH_2)_5Z''$

MST

(DMTr)danC $\mp$ T $\mp$ ibG $\mp$ anC $\mp$ T $\mp$ T $\mp$ T $\mp$ T $\mp$ $(CH_2)_5Z''$

1) $C_6H_5C(=O)NHOH$, DBU

2) $OH^{\ominus}$

3) $H^{\oplus}$

d[C–T–G–C–T–T–T–$(CH_2)_5Z''$]

EXEMPLE XIII

(DMTr)danC∓T∓ibG∓anC∓T∓T∓T∓CNEt

$H^{\oplus}$

(DMTr)danC–Ar danC∓T∓ibG∓anC∓T∓T∓T∓CNEt

MST

(DMTr)danC∓anC∓T∓ibG∓anC∓T∓T∓T∓CNEt

$NEt_3$

(DMTr)danC∓anC∓T∓ibG∓anC∓T∓T–Ar dT∓T∓ibG∓anC∓$(CH_2)_5$Z'

MST

(DMTr)danC∓anC∓T∓ibG∓anC∓T∓T∓T∓T∓ibG∓anC∓$(CH_2)_5$Z"

1) $C_6H_5C(=O)NHOH$,DBU

2) $OH^{\ominus}$

3) $H^{\oplus}$

d[C–C–T–G–C–T–T–T–T–G–C–$(CH_2)_5$Z"]

EXEMPLE XIV

(DMTr)danC∓anC∓T∓ibG∓anC∓T∓T–Ar dT∓anC∓ibG∓anC∓$(CH_2)_5$Z"

MST

(DMTr)danC∓anC∓T∓ibG∓anC∓T∓T∓T∓anC∓ibG∓anC∓$(CH_2)_5$Z"

1) $C_6H_6C(=O)NHOH$,DBU

2) $OH^{\ominus}$

3) $H^{\oplus}$

d[C–C–T–G–C–T–T–T–C–G–C–$(CH_2)_5$Z"]

EXEMPLE XV

(DMTr)dT∓anC∓ibG∓CNEt

$NEt_3$ $H^{\oplus}$

(DMTr)dT∓anC∓ibG–Ar dT∓anC∓ibG∓CNEt

MST

(DMTr)dT∓anC∓ibG∓T∓anC∓ibG∓CNEt

$NEt_3$

NEt$_3$

(DMTr)dT∓anC∓ibG+T∓anC∓ibG–Ar HO$(CH_2)_5$Z″

MST

(DMTr)dT∓anC∓ibG∓T∓anC∓ibG∓$(CH_2)_5$Z″

1) $C_6H_5C(=O)NHOH$,DBU

2) OH$^{\ominus}$

3) H$^{\oplus}$

d[T–C–G–T–C–G–$(CH_2)_5$Z″]

EXEMPLE XVI

(DMTr)dibG∓ibG∓anC∓bzA∓CNEt

NEt$_3$

(DMTr)dibG∓ibG∓anC∓bzA–Ar

(DMTr)dT∓anC∓ibG∓T∓anC∓ibG∓$(CH_2)_5$Z″

H$^{\oplus}$

dT∓anC∓zbG∓T∓anC∓ibG∓$(CH_2)_5$Z″

MST

(DMTr)dibG∓ibG∓anC∓bzA∓T∓anC∓ibG∓T∓anC∓ibG∓$(CH_2)_5$Z″

1) $C_6H_5C(=O)NHOH$,DBU

2) OH$^{\ominus}$

3) H$^{\oplus}$

d[G–G–C–A–T–C–G–T–C–G–$(CH_2)_5$Z″]

Tableau I

| Exemple | Système de solvants | Temps de rétention |
|---|---|---|
| I | A–C, gradient linéaire de 0 à 30% en C en 15 mn | 9 mn 29 sec |
| II | A–D, gradient linéaire de 0 à 30% en D en 15 mn | 4 mn |
| III | A–D, gradient linéaire de 0 à 30% en D en 15 mn | 4 mn |
| IV | A–D, gradient linéaire de 0 à 30% en D en 15 mn | 4 mn |
| VII | B–D, gradient linéaire de 0 à 50% en D en 15 mn | 8 mn 26 sec |
| VIII | B–D, gradient linéaire de 0 à 50% en D en 15 mn | 9 mn 30 sec |
| IX | B–D, gradient linéaire de 0 à 50% en D en 20 mn | 11 mn 34 sec |
| X | B–D, gradient linéaire de 0 à 50% en D en 20 mn | 14 mn 54 sec |
| XI | B–D, gradient linéaire de 0 à 50% en D en 20 mn | 15 mn |
| XII | B–D, gradient linéaire de 0 à 40% en D en 15 mn | 10 mn |
| XIII | B–D, gradient linéaire de 0 à 40% en D en 15 mn | 11 mn 17 sec |
| XIV | B–D, gradient linéaire de 0 à 40% en D en 15 mn | 11 mn 43 sec |
| XV | B–D, gradient linéaire de 0 à 50% en D en 15 mn | 11 mn 50 sec |
| XVI | B–D, gradient linéaire de 0 à 50% en D en 20 mn | 17 mn 30 sec |

HPLC, colonne polyanion HR 5/5 (Pharmacia), débit 1 ml/mn
A: $10^{-3}$ M phosphate de potassium (pH = 6)
B: $10^{-2}$ M phosphate de potassium (pH = 6)
C: 0,2 M phosphate de potassium (pH = 6)
D: 1 M phosphate de potassium (pH = 6)

Tableau II

| Exemple | Système de solvants | Temps de rétention |
|---|---|---|
| I | $A_1$–$B_2$, isocratique avec 65% de $B_2$ | 6 mn 38 sec |
| II | $B'_2$ | 6 mn |
| III | $A_1$–$B_2$, isocratique avec 94% de $B_2$ | 3 mn 24 sec |
| IV | $A_1$–$B_2$, isocratique avec 94% de $B_2$ | 3 mn 30 sec |
| V | $B_2$ | 10 mn |
| VI | $A_1$–$B_2$, isocratique avec 80% de $B_2$ | 6 mn 50 sec |
| VII | $A_1$–$B_2$, isocratique avec 65% de $B_2$ | 6 mn 41 sec |
| VIII | $A_1$–$B_2$, isocratique avec 65% de $B_2$ | 2 mn 42 sec |
| IX | $A_1$–$B_2$, isocratique avec 60% de $B_2$ | 6 mn 45 sec |
| X | $A_1$–$B_2$, isocratique avec 48% de $B_2$ | 4 mn 35 sec |
| XI | $A_1$–$B_2$, isocratique avec 48% de $B_2$ | 5 mn 36 sec |
| XII | $A_1$–$B_2$, isocratique avec 60% de $B_2$ | 7 mn |
| XIII | $A_1$–$B_2$, isocratique avec 48% de $B_2$ | 4 mn 19 sec |
| XIV | $A_1$–$B_2$, isocratique avec 48% de $B_2$ | 6 mn |
| XV | $A_2$–$B_2$, isocratique avec 65% de $B_2$ | 6 mn |
| XVI | $A_2$–$B_2$, isocratique avec 50% de $B_2$ | 5 mn |

HPLC, colonne Lichrosorbe RP-18 (Merck); débit: 1,2 ml/mn
$A_1$: $CH_3CN$, AAc, eau (18:88:794, v/v)
$A_2$: $CH_3CN$, AAc, eau (90:88:722, v/v)
$B_2$: $CH_3CN$, AAc, eau (216:79:605, v/v)
$B'_2$ $CH_3CN$, TEAc, eau (216:79:605, v/v)
AAc: solution aqueuse d'acétate d'ammonium à 10% en poids (pH = 5,9)
TEAc: solution aqueuse d'acétate de triéthylammonium à 10% en poids (pH = 5,9)

EXEMPLE XVII

Action des composés I sur l'expression du gène 32 du phage T4

Le tableau III donne la séquence du gène 32 du phage T4 au voisinage du site d'initiation de la traduction de l'ARN messager. Le brin supérieur (codant) correspond à la séquence de cet ARN messager (en remplaçant T par U). La traduction de l'ARN messager est régulée par le produit du gène 32 (protéine 32 ou gp 32). Les oligonucléotides de formule générale I qui ont été synthétisés et décrits dans le brevet français no 8 301 223 sont représentés dans la partie inférieure. Ils sont complémentaires de séquences répétées situées en amont de la séquence «Shine et Dalgarno» responsable de la fixation des ribosomes. Les trois composés I synthétisés comportent 5, 10 ou 15 nucléotides; ils sont liés par leur groupement 3'-phosphate au groupe méthoxy-2-chloro-6-(ω-pentylamino)-9-acridine, schématisée par $(CH_2)_5Acr$.

L'effet de ces oligonucléotides sur la synthèse de la protéine gp 32 a été étudié dans un système de transcription-traduction in vitro préparé à partir de la souche D10 d'E. coli, appelé S-30. Ce système comporte les macromolécules et les organites nécessaires à la transcription et à la traduction d'un ADN exogène comportant les sites de régulation pour ces processus. L'ADN utilisé est le plasmide pKH13 décrit par H.M. KRISCH et B. ALLET (1982) Proc. Nat. Acad. Sci. 79, p. 4937–4941. Ce plasmide construit à partir de

TABLEAU III

GENE 32 – PHAGE T4

SEQUENCES REPETEES

SHINE DALGARNO

MET PHE LYS

5'G C ACCAACTGT T AATTAAATT A AATTAAAAA G GAAATAAAA A TGTTTAAA3'
3'C G TGGTTGACA A TTAATTTAA T TTAATTTTT C CTTTATTTT T ACAAATTT5'

–40 –30 –20 –10 –1–

$[Acr](CH_2)_5$ A A T T $T_{5'}$
$[Acr](CH_2)_5$ A A T T T A A T T $T_{5'}$
$[Acr](CH_2)_5$ A A T T T A A T T T A A T T $T_{5'}$
} OLIGONUCLEOTIDES SYNTHETIQUES

pBR322 comporte une insertion de 2000 nucléotides provenant du génome de T4, dont le gène 32 portant une mutation ambre en position 116 de la chaîne protéique. Celui-ci dirige la syntèse d'une protéine raccourcie (gp'32) par rapport à la protéine 32 normale. Cette protéine raccourcie ne régule pas sa propre synthèse contrairement à la protéine 32 complète. Dans le système S-30, la synthèse de protéines commandée par pKH13 est effectuée en présence de méthionine $^{35}S$; l'analyse est faite sur gel de polyacrylamide à 15%, révélé par autoradiographie. On constate que l'oligonucléotide de même séquence ne portant pas l'agent d'intercalation n'a pas d'effet sur la synthèse de pg'32. Un 8-mère $(T-)_8(CH_2)_6Acr$) dont la séquence n'est pas complémentaire d'une séquence du gène 32 n'a aucun effet sur la synthèse de gp' 32. Le 15-mère couplé à l'acridine inhibe la synthèse de la protéine gp'32. Le même oligonucléotide sans intercalant n'a pas d'activité. Les résultats obtenus démontrent qu'il est possible d'inhiber l'expression d'un gène défini par bloquage de sa traduction à l'aide d'oligonucléotides liés à un agent d'intercalation lorsque la séquence de l'oligonucléotide est complémentaire d'une séquence portée par l'ARN messager.

EXEMPLE XVIII

Action des composés VI, VII, VIII sur la transcription du gène de la β-lactamase

Le tableau IV donne la séquence du gène bla de la β-lactamase porté par le plasmide pBR322 et le transposon Tn3 et qui est responsable de la résistance des bactéries aux antibiotiques de la famille des β-lactames (pénicilline, ampicilline . . . ). Le gène bla porté par le plasmide pBR322 provient originellement d'une bactérie du type Salmonella paratyphi B.

Le tableau IV indique le site d'initiation de la transcription du gène bla (position + 1 sur la séquence). En dessous se trouve indiquée la région supposée ouverte par le RNA polymérase lorsqu'elle se fixe sur le promoteur du gène bla. Dans la partie inférieure du tableau IV sont rappelés les trois oligonucléotides liés à l'agent d'intercalation Z'' (ici représenté par $(CH_2)_5Acr$).

La transcription du gène bla par la RNA polymérase d'E. coli (produit Boehringer) a été suivie à la fois sur le plasmide pBR322 et sur un fragment de 750 paires de bases contenant le promoteur et une partie importante du gène bla. Ce fragment a été obtenu par action des enzymes de restriction PstI et EcoRI. En utilisant trois nucléotides triphosphates (ATP, GTP et CTP) en excès (100 fois) par rapport à l'UTP marqué par un $^{32}P$ en position α, on obtient des transcrits de courte taille marqués radioactivement. Ces transcrits sont séparés sur gels de polyacrylamide en présence d'urée 7 M et révélés par autoradiographie. Pour assurer une initiation spécifique de la transcription, un dinucléoside monophosphate UpG peut être ajouté au système de transcription.

L'étude de l'effet des composés des exemples VII et VIII sur la transcription du gène bla a permis de constater que ces deux composés bloquent la transcription du gène à 37 °C. L'effet est spécifique: en effectuant l'étude sur le plasmide pBR322 qui porte plusieurs gènes, seulement une partie des transcrits est affectée par les composés des exemples VII et VIII, ceux qui sont initiés au niveau du promoteur du gène bla. L'effet observé requiert la présence de l'agent d'intercalation; un oligonucléotide de même séquence que VII et VIII n'a pas d'effet sur l'initiation de la transcription du gène bla. Une longueur minimum de l'oligonucléotide est nécessaire: le composé VI (trois nucléotides) n'a aucun effet.

Ces expériences démontrent qu'il est possible de bloquer sélectivement l'expression d'un gène de résistance à un antibiotique en inhibant l'initiation de la transcription de ce gène. La spécificité est obtenue par synthèse d'une séquence d'oligonucléotide complémentaire du brin non codant de l'ADN du voisinage du site d'initiation de la transcription (en amont).

TABLEAU IV

GENE B1a (Tn3) – REGION D'INITIATION DE LA TRANSCRIPTION

*bla* mRNA

−10 region | +1 | Met Ser

TGA GACAATA ACCCTGATAAATGCTTCAATAATATTGAAAAAGGAAGAGTATGAGT
ACT CTGTTAT TGGGACTATTTACGAAGTTATTATAACTTTTTCCTTCTCAGACTCA

REGION OUVERTE PAR LA RNA POLYMERASE

−10 −1+1

5'—AGA CAATAACCCTGAT AAA—3'
3'—TCT GTTATTGGGACTA TTT—5'

TGA$(CH_2)_5$Acr
CCCTGA$(CH_2)_5$Acr
TAACCCTG$(CH_2)_5$Acr
} OLIGONUCLEOTIDES SYNTHETIQUES

EXEMPLE XIX

Activités inhibitrices de la multiplication du virus SV40

Le virus SV40 est caractérisé par un génome d'ADN circulaire à double brin. La réplication de l'ADN démarre dans un site déterminé du génome, appelé «origine de réplication», constitué d'environ une centaine de nucléotides. Une séquence de 17 paires de bases A.T., et plus particulièrement un double brin polydA ·polydT, se trouvent à l'intérieur de l'origine de réplication. L'existence de cette séquence polydA · polydT permet d'envisager le blocage de l'initiation de la réplication virale par le biais d'un oligothymidilate couplé à une molécule intercalante.

L'effet de différents composés a été examiné vis-à-vis de la multiplication du virus SV40 en cultures de cellules rénales de singe (cellules CV1):

$HO(CH_2)_5Acr$, T–T–T–T–Et, T–T–T–T–$(CH_2)_5Acr$,
T–T–T–T–T–T–T–T–Et et
T–T–T–T–T–T–T–T–$(CH_2)_6ACr$,
(les composés $(T–)_4(CH_2)_5Acr$ et $(T–)_8(CH_2)_6Acr$ sont
obtenues selon le brevet français no 8 301 223).

Des monocouches de cellules CV1 obtenues après culture à 37 °C, 10% $CO_2$, en boîtes de Pétri de 9 $cm^2$, sont vidées de leur milieu de culture (milieu Eagle modifié par Dulbecco contenant 7% de sérum de veau fétal, 2 mM glutamine, 0,22% de bicarbonate sodique, 40 µg/ml de pénicilline et 40 µg/ml de streptomycine) et inoculées avec 0,2 ml d'une suspension de SV40. La dilution de virus dans un tampon phosphate isotonique a été choisie de façon telle à obtenir dans les cultures témoins environ 75% de destruction cellulaire (75% d'effet cytopathogène) après 3 jours d'incubation. Après 1 heure de contact à 37 °C, l'inoculum est retiré et 2 ml du même milieu contenant ou non les produits à étudier sont ajoutés. Après 3 jours d'incubation à 37 °C, sous une atmosphère à 10% de $CO_2$, les couches cellulaires sont examinées à l'aide d'un microscope inversé (25 × 10). Des cultures de cellules non infectées par le virus et traitées avec les produits ont été aussi examinées pour déceler des effets cytotoxiques éventuels. Les pourcentages de destruction cellulaire dans les cultures infectées (effet cytopathogène) et dans les cultures non infectées (effet cytotoxique des produits) sont indiqués dans le tableau V.

Tableau V

| Produit | Concentration en µg/ml | Pourcentage de destruction cellulaire — Cultures infectées par SV40 (effet cytopathogène) | Pourcentage de destruction cellulaire — Cultures non infectées (effet toxique) |
|---|---|---|---|
| 0 cultures témoins | 0 | 75 | 0 |
| $Acr(CH_2)_5OH$ | 0,25 | 75 | 0 |
| | 0,5 | 50 | 0 |
| | 1,0 | 75 | 20 (Toxique) |
| | 2,0 | 100 | 100 (Toxique) |
| $(T–)_4(CH_2)_5Acr$ | 20 | 44 | 0 |
| | 40 | 33 | 0 |
| | 80 | 21 | 0 |
| $(T–)_4Et$ | 20 | 75 | 0 |
| | 40 | 70 | 0 |
| | 80 | 64 | 20 |
| $(T–)_8(CH_2)_6Acr$ | 20 | 50 | 0 |
| | 40 | 0 | 0 |
| | 80 | 0 | 0 |
| $(T–)_8Et$ | 20 | 50 | 0 |
| | 40 | 50 | 0 |
| | 80 | 50 | 0 (Toxique) |

Des faibles inhibitions de la multiplication virale (jugée par l'effet cytopathogène) sont obtenues avec les oligothymidilates non couplés à de l'acridine. Les produits $(T–)_4Et$ et $(T–)_8$ et donnent respectivement 64% et 50% d'effet cytopathogène par rapport à 75% dans les cultures non traitées. En revanche, les deux oligothymidilates comportant le groupe acridine $(T–)_4(CH_2)_5Acr$ et $(T–)_8(CH_2)_6Acr$ à la concentration de 40 µg/ml diminuent, $(T–)_4(CH_2)_5Acr$, ou empêchent complètement, $(T–)_8(CH_2)_6Acr$, l'effet cytopathogène. A 80 µg/ml cet effet inhibiteur est maintenu ou accru sans qu'on puisse constater, dans les cultures non infectées, les effets cytotoxiques observés dans le cas des oligonucléotides non couplés à l'acridine. L'intercalant $Acr(CH_2)_5OH$ n'exerce pas d'effet inhibiteur significatif sur la multiplication virale à 0,25 et à 0,5 µg/ml. A 1 et à 2 µg/ml, ce produit se révèle toxique pour des cellules non infectées. En vue d'apprécier la sélectivité de son action anti-SV40, le produit $(T-)_4(CH_2)_5Acr$ a été examiné aussi vis-à-vis de la multiplication du virus grippal A/Victoria ($H_3N_2$) (en cultures de cellules rénales MDCK) et des virus de l'herpès type 1 du rhinovirus 1B (en cultures de cellules embryonnaires $MRC_5$). Aucune inhibition de l'effet cytopathogène n'a été constatée. Le produit $(T-)_8(CH_2)_6Acr$ a été examiné également pour son effet inhibiteur éventuel de la multiplication du virus grippal et dans ce même test il a été trouvé inactif. Ces résultats indiquent que des oligothymidilates couplés à une acridine exercent des activités inhibitrices sélectives vis-à-vis de la réplication d'un virus (SV40) contenant dans son origine de réplication des séquences complémentaires correspondantes.

EXEMPLE XX

Activités inhibitrices de la multiplication de virus grippaux

Le génome des virus grippaux consiste en ARNs en simple brin, segmenté en 8 fragments. Sauf dans le cas de l'ARN qui code pour la neuraminidase (bis cistronique), les autres ARNs codent chacun pour un seul gène viral. La structure primaire de ces ARNs en position 3' est bien conservée parmi chacun de ces fragments et parmi les différents variants antigènes de virus grippal du même type (A, B, C) qui ont été examinés. Cette séquence est: UCG($^{U}_{C}$)UUUCGUCC pour les 12 premiers nucléotides. De ce fait, découle la possibilité d'interférer avec la réplication de plusieurs souches de virus grippal par le biais d'oligonucléotides complémentaires de cette séquence et couplés à un intercalant (acridine).

L'effet des composés des exemples IX, X et XI a été étudié vis-à-vis de la multiplication des deux virus grippaux A/Victoria et A/Philippines (tous les deux $H_3N_2$) en cultures de cellules MDCK. Des plaques (24 puits, 2 $cm^2$) contenant des monocouches cellulaires ont été vidées de leur milieu de culture (milieu MEM avec sels de Earle, contenant 10% de sérum de veau fétal, 0,22% de bicarbonats sodique, 40 µg/ml de pénicilline et 40 µg/ml de streptomycine) et inoculées avec 0,1 ml du produit à tester, 0,1 ml d'une suspension virale (à une dilution choisie de façon telle à obtenir après 3 jours de culture une destruction cellulaire – effet cytopathogène – complète) et 0,8 ml de milieu MEM (sels de Earle) contenant 5 µg/ml de trypsine, 0,22% de bicarbonate sodique, 40 µg/ml de pénicilline et 40 µg/ml de streptomycine.

Des cultures non infectées par le virus ont été utilisées pour rechercher les effets cytotoxiques éventuels de ces produits. Les plaques ont été incubées à 37 °C, sous une atmosphère à 10% $CO_2$. Après 3 jours d'incubation, 1 ml de glutaraldéhyde à 5% est ajouté par puits. 20 minutes après, les puits sont vidés et les couches cellulaires fixées sont colorées avec une solution éthanolique (14%) de crystal violet à 0,1% pendant 20 minutes. Après rinçage à l'eau, la destruction des couches est évaluée au microscope et exprimée en pourcentage (0%: couche intacte; 100%: couche entièrement détruite).

Les pourcentages de destruction cellulaire dans les cultures infectées (effet cytopathogène) et dans les cultures non infectées (effet cytotoxique) sont indiqués dans le tableau VI.

Tableau VI

| | | Pourcentage de destruction cellulaire | | |
|---|---|---|---|---|
| Produit | Concentration finale en µg/ml | Cultures infectées (effet cytopathogène) par A/Victoria ($H_3N_2$) | A/Philippines ($H_3N_2$) | Cultures non infectées (effet cytotoxique) |
| $Acr(CH_2)_5OH$ | 0,25 | 100 | – | 0 |
| | 0,5 | 100 | – | 20 (Toxique) |
| | 1,0 | 100 | – | 50 (Toxique) |
| A-G-C-A-A-A-A-G-C-A-G-$(CH_2)_5$Acr XI | 75 | 100 | 100 | 0 |
| | 150 | 100 | 100 | 0 |
| | 300 | 100 | 100 | 20 (Toxique) |
| A-A-A-G-C-A-G-$(CH_2)_5$Acr IX | 75 | 100 | 100 | 0 |
| | 150 | 50 | 70 | 0 |
| | 300 | 5 | 30 | 0 |
| A-G-C-G-A-A-A-G-C-A-G-$(CH_2)_5$Acr X | 75 | 100 | 100 | 0 |
| | 150 | 100 | 100 | 0 |
| | 300 | 100 | 75 | 0 |

Les résultats obtenus indiquent que le produit IX exerce des effets inhibiteurs vis-à-vis de la multiplication des deux virus grippaux à des concentrations (150 et 300 µg/ml) qui sont dépourvues de cytotoxicité sur des cultures cellulaires non infectées. Les produits X et XI sont presque totalement inactifs (sauf une faible inhibition sur le virus A/Philippines à 300 µg/ml pour le produit X). Comme cela est indiqué dans le tableau VI, le composé $Acr(CH_2)OH$ est dépourvu d'activité. Ces résultats indiquent que des oligonucléotides couplés à un intercalant, par exemple le composé IX, peuvent être utilisés comme inhibiteurs de la multiplication de virus grippaux.

Pénétration dans les cellules en culture

Des cellules en culture ont été incubées en présence des oligonucléotides liés de façon covalente au dérivé intercalant de l'acridine. La pénétration a été suivie en mesurant la fluorescence de l'intercalant de façon qualitative (observation et prise de clichés en microscopie de fluorescence) et de façon quantitative (microspectrofluorimétrie). La fluorescence verte de $(T-)_8(CH_2)_6Acr$ est clairement visible dans les cellules de Lewis (tumeur humaine du poumon), dans les cellules EL2 de souris ou dans des cellules CV1 de rein de singe. La pénétration a lieu dans des temps très courts. En 15 minutes, la fluorescence intracellulaire a atteint son maximum. Le spectre de fluorescence enregistré par microspectrofluorimétrie sur une cellule isolée est bien celui attendu pour le dérivé de l'acridine utilisés lié à son oligodésoxynucléotide.

ABRÉVIATIONS

Dans la description, on utilise la représentation condensée des nucléotides suivante:

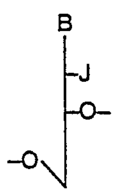

qui correspond à la formule développée:

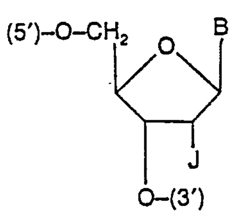

sur laquelle ont été mentionnées les extrémités (3') et (5').

Dans la présente description, les abréviations ci-après sont utilisées

| | |
|---|---|
| A | 2'-déoxyadénosine |
| C | 2'-déoxycitidine |
| G | 2'-déoxyguanosine |
| bzA | N-benzoyl-2'-déoxyadénosine |
| anC | N-anisoyl-2'-déoxycytidine |
| ibG | N-isobutyryl-2'-déoxyguanosine |
| ODN | oligodésoxyribonucléotide |
| Ar | parachlorophyle |
| CNEt | β-cyanoéthyle |
| Me | méthyle |
| Et | éthyle |
| DMTr | diméthoxytrityle |
| MST | mésitylènesulfonyltétrazolide |
| DMT | diméthyl-1,5-tétrazole |
| T | thymidine |

Z': 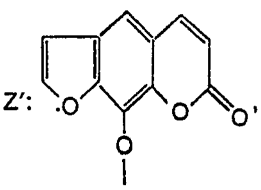, Z'': 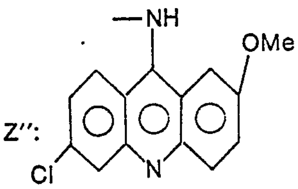

Z''': 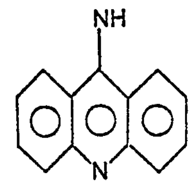

| | |
|---|---|
| TEA | triéthylamine |
| CCM | chromatographie sur couche mince |

Dans les formules (DMTr)dbzA∓anC∓ibG∓T–Ar, la liaison phosphodiester est représentée par un trait et la liaison phosphotriester est représentée par le symbole (∓), chaque phosphate est protégé par le groupe p-chlorophényle (Ar).

**Revendications pour les Etats contractants: BE, CH, DE, FR, GB, IT, LI, LU, NL, SE**

1. A titre de médicaments, des composés oligonucléotides liés à un agent intercalant, constitués par un oligonucléotide ou un oligodésoxynucléotide de constitué d'un enchaînement de nucléotides naturel ou modifié complémentaire d'une séquence d'acide nucléique, sur lequel se trouve fixé par une liaison covalente un groupe d'intercalation, ladite séquence d'acide nucléique constituant tout ou partie d'une séquence impliquée dans l'initiation, la propagation ou la terminaison de la réplication d'un acide nucléique, de la transcription et/ou de la traduction d'un ou plusieurs gènes.

2. A titre de médicaments selon la revendication 1, des composés caractérisés en ce que l'oligonucléotide ou l'oligodésoxynucléotide est complémentaire d'une séquence assurant la réplication ou le développement d'un virus, d'une bactérie ou d'un parasite.

3. A titre de médicaments selon la revendication 2, des composés caractérisés en ce que la séquence d'oligonucléotides ou d'oligodésoxynucléotides est complémentaire d'une région de régulation, d'initiation ou de propagation de la réplication.

4. A titre de médicaments selon la revendication 1, des composés caractérisés en ce que la séquence d'oligonucléotides ou d'oligodésoxynucléotides est complémentaire d'une séquence

d'acide nucléique assurant l'expression d'un gène codant pour un facteur de résistance aux antibiotiques, aux antiviraux ou aux antiparasitaires.

5. A titre de médicaments selon l'une des revendications 2 et 3, des composés caractérisés en ce que le virus est le virus de la grippe ou le virus de l'herpès.

6. A titre de médicaments selon la revendication 1, des composés caractérisés en ce que la séquence d'oligonucléotides ou d'oligodésoxynucléotides est complémentaire d'une séquence d'acide nucléique assurant l'expression d'un gène oncogène.

7. A titre de médicaments selon l'une des revendications 1 à 6, des composés de formule:

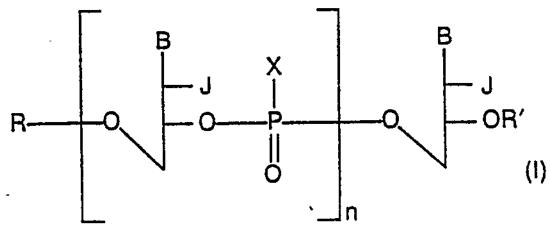

(I)

dans laquelle:

les radicaux B peuvent être identiques ou différents et représentent chacun une base d'un acide nucléique naturelle ou modifiée;

les radicaux X, qui peuvent être identiques ou différents, représentent chacun un oxoanion $O^{\ominus}$, un thioanion $S^{\ominus}$, un groupe alkyle, un groupe alcoxy, aryloxy, alcoxy ou alkyle substitué par un hétérocycle azoté, un groupe aminoalkyle, un groupe aminoalcoxy, un groupe thioalkyle ou un groupe –Y–Z;

R et R′, qui peuvent être identiques ou différents, représentent chacun un atome d'hydrogène ou un groupement –Y–Z dans lequel:

Y représente un radical alcoylène droit ou ramifié –alk– ou un radical

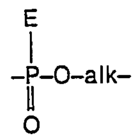

dans lequel E peut avoir les mêmes significations que X excepté Y–Z; ou bien un radical –Y″–O–Y′ où Y″ et Y′ peuvent avoir les significations données pour Y; et

Z est un radical correspondant à un agent d'intercalation; l'un seulement de X, R et R′ est un groupe Y–Z;

J représente un atome d'hydrogène ou un groupe hydroxy;

n est un nombre entier y compris O.

8. A titre de médicaments selon la revendication 7, des composés caractérisés en ce que l'agent d'intercalation Z est choisi parmi les composés polycycliques ayant une configuration plane.

9. A titre de médicaments selon la revendication 8, des composés caractérisés en ce que Z est choisi parmi l'acridine, la furocoumarine ou l'ellipticine.

10. A titre de médicaments selon l'une des revendications 1 à 9, des composés caractérisés en ce que le radical B est un radical correspondant à la thymine, l'adénine, la 2-aminoadénine et ses dérivés substitués, par exemple sur le $N^6$, par un groupe aminoalkylène ou par un groupe azidophénylalkylène, la cytosine, la guanine ou la guanine substituée sur le $O^6$, par exemple par un groupe (ω-alkylène)-9-acridine, la 8-(ω-aminoalkyl)-aminoadénine et ses dérivés substitués sur le $NH_2$ en ω par un groupe acridine; l'uracile, la 5-bromo-uracile, le 8-azidoadénine ou un dérivé photoactivable des bases.

11. A titre de médicaments selon l'une des revendications 1 à 10, des composés caractérisés en ce que le radical Z est choisi parmi:

Z′ 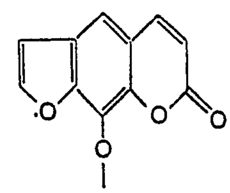

Z″ 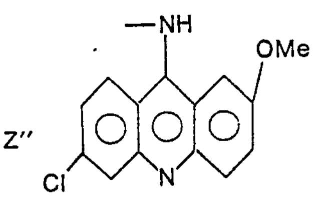

Z‴ 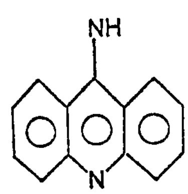

12. A titre de médicaments selon l'une des revendications 1 à 11, des composés caractérisés en ce que B est choisi parmi, T, G, A, C et U; X est choisi parmi $O^-$, méthyle, éthyle, propyle, méthoxy, éthoxy, propoxy et J = H.

13. A titre de médicaments selon la revendication 1, des composés caractérisés en ce que les composés sont choisis parmi:

$(T-)_n(CH_2)_nZ$

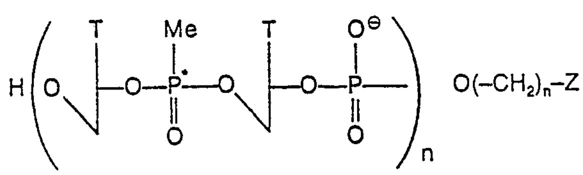

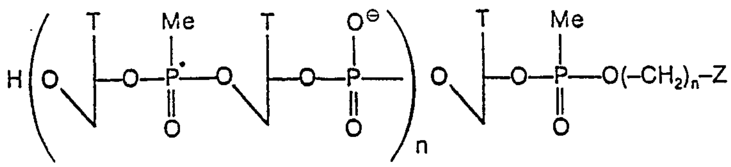

d[T–G–A–$(CH_2)_n$Z]

d[C–C–C–T–G–A–$(CH_2)_n$Z]

d[T–A–A–C–C–C–T–G–A–$(CH_2)_n$Z]

d[A–A–A–G–C–A–G–$(CH_2)_n$Z]

d[A–G–C–G–A–A–A–G–C–A–G–$(CH_2)_n$Z]

d[A–G–C–A–A–A–A–G–C–A–G–$(CH_2)_n$Z]

d[C–T–G–C–T–T–T–$(CH_2)_n$Z]

d[C–C–T–G–C–T–T–T–T–G–C–$(CH_2)_n$Z]

d[C–C–T–G–C–T–T–T–T–G–C–$(CH_2)_n$Z]

d[C–C–T–G–C–T–T–T–C–G–C$(CH_2)_n$Z]

d[T–C–G–T–C–G–$(CH_2)_n$Z]

d[G–G–C–A–T–C–G–T–C–G–$(CH_2)_n$Z]

dans lesquels Z est un agent intercalant.

14. Composition pharmaceutique, caractérisée en ce qu'elle comporte à titre de principe actif au moins un médicament selon l'une des revendications 1 à 13 et un support acceptable en pharmacie.

15. Composition pharmaceutique selon la revendication 14, caractérisée en ce qu'elle comporte, en outre, un antibiotique, un agent antiparasitaire, un agent antiviral ou un agent antitumoral.

**Revendications pour l'Etat contractant: AT**

1. Procédé de fabrication de médicaments consistant en des composés oligonucléotides liés à un agent intercalant, constitués par un oligonucléotide ou un oligodésoxynucléotide constitué d'un enchaînement de nucléotides naturel ou modifié complémentaire d'une séquence d'acide nucléique, caractérisé en ce qu'on fixe par une liaison covalente un groupe d'intercalation sur ladite séquence d'acide nucléique constituant tout ou partie d'une séquence impliquée dans l'initiation, la propagation ou la terminaison de la réplication d'un acide nucléique, de la transcription et/ou de la traduction d'un ou plusieurs gènes.

2. Procédé de fabrication de médicaments selon la revendication 1, caractérisé en ce que l'oligonucléotide ou l'oligodésoxynucléotide est complémentaire d'une séquence assurant la réplication ou le développement d'un virus, d'une bactérie ou d'un parasite.

3. Procédé de fabrication de médicaments selon la revendication 2, caractérisé en ce que la séquence d'oligonucléotides ou d'oligodésoxynucléotides est complémentaire d'une région de régulation, d'initiation ou de propagation de la réplication.

4. Procédé de fabrication de médicaments selon la revendication 1, caractérisé en ce que la séquence d'oligonucléotides ou d'oligodésoxynucléotides est complémentaire d'une séquence d'acide nucléique assurant l'expression d'un gène codant pour un facteur de résistance aux antibiotiques, aux antiviraux ou aux antiparasitaires.

5. Procédé de fabrication de médicaments selon l'une des revendications 2 et 3, caractérisé en ce que le virus est le virus de la grippe ou le virus d'herpès.

6. Procédé de fabrication de médicaments selon la revendication 1, caractérisé en ce que la séquence d'oligonucléotides ou d'oligodésoxynu-

cléotides est complémentaire d'une séquence d'acide nucléique assurant l'expression d'un gène oncogène.

7. Procédé de fabrication de médicaments selon l'une des revendications 1 à 6, de formule:

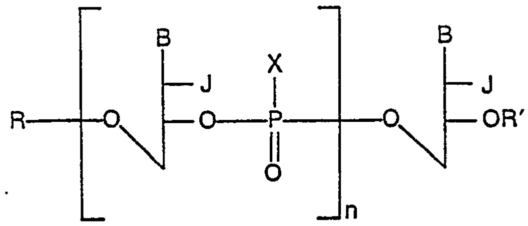

(I)

dans laquelle:

les radicaux B peuvent être identiques ou différents et représentent chacun une base d'un acide nucléique naturelle ou modifiée; les radicaux X, qui peuvent être identiques ou différents, représentent chacun un oxoanion $O^{\ominus}$, un tioanion $S^{\ominus}$, un groupe alkyle, un groupe alcoxy, aryloxy, alcoxy ou alkyle substitué par un hétérocycle azoté, un groupe aminoalkyle, un groupe aminalcoxy, un groupe thioalkyle ou un groupe –Y–Z;

R et R′, qui peuvent être identiques ou différents, représentent chacun un atome d'hydrogène ou un groupement –X–Z dans lequel;

Y représente un radical alcoylène droit ou ramifié –alk– ou un radical

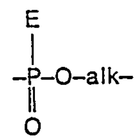

dans lequel E peut avoir les mêmes significations que X excepté Y–Z; ou bien un radical –Y″–O–Y′ où Y″ et Y′ peuvent avoir les significations données pour Y; et

Z est un radical correspondant à un agent d'intercalation; l'un seulement de X, R et R′ est un groupe Y–Z;

J représente un atome d'hydrogène ou un groupe hydroxy;

n est un nombre entier y compris O.

8. Procédé de fabrication de médicaments selon la revendication 7, caractérisé en ce que l'agent d'intercalation Z est choisi parmi les composés polycycliques ayant une configuration plane.

9. Procédé de fabrication de médicaments selon la revendication 8, caractérisé en ce que Z est choisi parmi l'acridine, la furocoumarine ou l'ellipticine.

10. Procédé de fabrication de médicaments selon l'une des revendications 1 à 9, caractérisé en ce que le radical B est un radical correspondant à la thymine, l'adénine, la 2-aminoadénine et ses dérivés substitués, par exemple sur le $N^6$, par un groupe aminoalkylène ou par un groupe azidophénylalkylène, la cytosine, la guanine ou la guanine substituée sur le $O^6$, par exemple par un groupe (ω-alkylène)-9-acridine, la 8-(ω-aminoalkyl)-aminoadénine et ses dérivés substitués sur le $NH_2$ en ω par un groupe acridine; l'uracile, la 5-bromo-uracile, le 8-azidoadénine ou un dérivé photoactivable des bases.

11. Procédé de fabrication de médicaments selon l'une des revendications 1 à 10, caractérisé en ce que le radical Z est choisi parmi:

Z′ 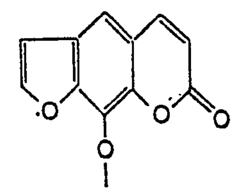

Z″ 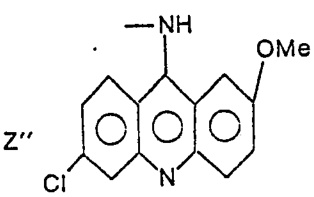

Z‴ 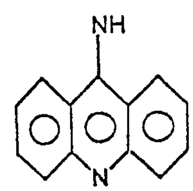

12. Procédé de fabrication de médicaments selon l'une des revendications 1 à 11, caractérisé en ce que B est choisi parmi T, G, A, C et U; X est choisi parmi $O^-$, méthyle, éthyle, propyle, méthoxy, éthoxy, propoxy et J = H.

13. Procédé de fabrication de médicaments selon la revendication 1, caractérisé en ce que les composés sont choisis parmi:

$(T-)_n(CH_2)_nZ$

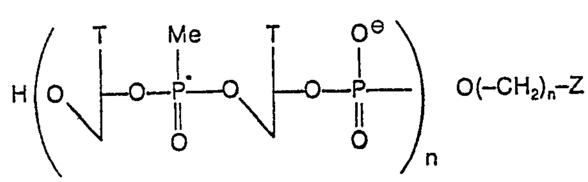

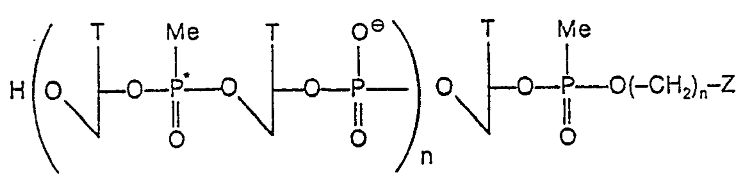

d[T–G–A–$(CH_2)_n$Z]

d[C–C–C–T–G–A–$(CH_2)_n$Z]

d[T–A–A–C–C–C–T–G–A–$(CH_2)_n$Z]

d[A–A–A–G–C–A–G–$(CH_2)_n$Z]

d[A–G–C–G–A–A–A–G–C–A–G–$(CH_2)_n$Z]

d[A–G–C–A–A–A–A–G–C–A–G–$(CH_2)_n$Z]

d[C–T–G–C–T–T–T–$(CH_2)_n$Z]

d[C–C–T–G–C–T–T–T–T–G–C–$(CH_2)_n$Z]

d[C–C–T–G–C–T–T–T–T–G–C–$(CH_2)_n$Z]

d[C–C–T–G–C–T–T–T–C–G–C$(CH_2)_n$Z]

d[T–C–G–T–C–G–$(CH_2)_n$Z]

d[G–G–C–A–T–C–G–T–C–G–$(CH_2)_n$Z]

dans lesquels Z est un agent intercalant.

14. Procédé de préparation d'une composition pharmaceutique, caractérisé en ce qu'on adjoint à titre de principe actif au moins un médicament préparé selon l'une des revendications 1 à 13 et un support acceptable en pharmacie.

15. Procédé de préparation d'une composition pharmaceutique selon la revendication 14, caractérisé en ce qu'on lui adjoint en outre un antibiotique, un agent antiparasitaire, un agent antiviral ou un agent antitumoral.

**Claims for the Contracting States: BE, CH, DE, FR, GB, IT, LI, LU, NL, SE**

1. Drugs, in the form of oligonucleotide compounds bonded to an intercalating agent and comprising an oligonucleotide or an oligodesoxynucleotide made up of a chain of natural or modified nucleotides and complementary with a nucleic acid sequence to which an intercalating group is attached by a covalent bond, the nucleic acid sequence constituting all or part of a sequence involved in initiation, propagation or termination of the replication of a nucleic acid and of transcription and/or translation of one or more genes.

2. Drugs according to claim 1, in the form of compounds characterised in that the oligonucleotide or oligodesoxynucleotide is complementary with a sequence for replicating or developing a virus or bacterium or parasite.

3. Drugs according to claim 2, in the form of compounds characterised in that the sequence of oligonucleotides or oligodesoxynucleotides is complementary with a region for regulation, initiation or propagation of replication.

4. Drugs according to claim 1, in the form of compounds characterised in that the sequence of oligonucleotides or oligodesoxynucleotides is complementary with a nucleic acid sequence for expressing a gene which codes for a factor giving resistance to antibiotics or antiviral agents or antiparasitic agents.

5. Drugs according to claim 2 or 3, in the form of compounds characterised in that the virus is the influenza virus or the herpes virus.

6. Drugs according to claim 1, in the form of compounds characterised in that the sequence of oligonucleotides or oligodesoxynucleotides is complementary with a nucleic acid sequence for expressing an oncogenic gene.

7. Drugs according to any of claims 1 to 6, in the form of compounds having the formula:

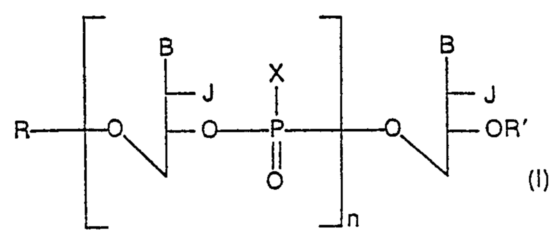

(I)

in which:

the radicals B can be identical or different and each denote a natural or modified nucleic acid base,

the radicals X, which can be identical or different, each represent an oxoanion $O^{\ominus}$ or a thioanion $S^{\ominus}$ or an alkyl group or an alkoxy, aryloxy, alkoxy or alkyl group substituted by a nitrogen-containing heterocycle, or an aminoalkyl group or an aminoalkoxy group or a thioalkyl group or a –Y–Z group;

R and R′, which can be identical or different, each represent a hydrogen atom or a –Y–Z group in which:

Y represents a straight-chain or branched alkylene radical –alk– or a radical

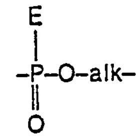

in which E can have the same meanings as X except Y–Z; or a radical –Y″–O–Y′ where Y″ and Y′ can have the meanings given for Y,

Z is a radical corresponding to an intercalating agent; only one out of X, R and R′ is a Y–Z group;

J represents a hydrogen atom or a hydroxy group, and

n is an integer including O.

8. Drugs according to claim 7, in the form of compounds characterised in that the intercalating agent Z is chosen from among polycyclic compounds having a plane configuration.

9. Drugs according to claim 8, in the form of compounds characterised in that Z is chosen from among acridine, furocoumarin or ellipticine.

10. Drugs according to any of claims 1 to 9, in the form of compounds characterised in that the radical B is a radical corresponding to thymine, adenine, 2-aminoadenine and derivatives thereof substituted e.g. on the $N^6$ by an aminoalkylene group or by an azidophenylalkylene group, cytosine, guanine or guanine substituted on the $O^6$ e.g. by an (ω-alkylene)-9-acridine group, or 8-(ω-aminoalkyl)-aminoadenine and derivatives thereof substituted on the $NH_2$ at ω by an acridine group; uracil, 5-bromo-uracil, 8-azidoadenine or a photoactivatable derivative of the bases.

11. Drugs according to any of claims 1 to 10, in the form of compounds characterised in that the radical Z is chosen from among:

Z′ 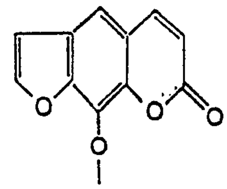

Z″ 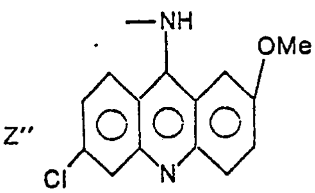

Z‴ 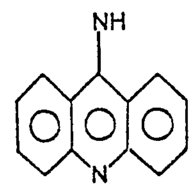

12. Drugs according to any of claims 1 to 11, in the form of compounds characterised in that B is chosen from among T, G, A, C and U; X is chosen from among $O^-$, methyl, ethyl, propyl, methoxy, ethoxy and propoxy, and J = H.

13. Drugs according to claim 1, in the form of compounds characterised in that the compounds are chosen from among:

$(T-)_n(CH_2)_nZ$

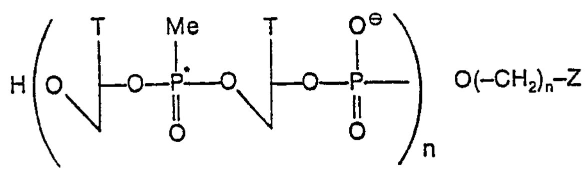

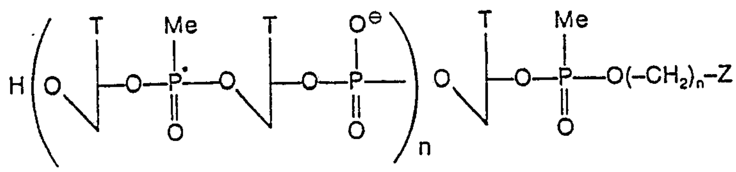

$d[T-G-A-(CH_2)_nZ]$

$d[C-C-C-T-G-A-(CH_2)_nZ]$

$d[T-A-A-C-C-C-T-G-A-(CH_2)_nZ]$

$d[A-A-A-G-C-A-G-(CH_2)_nZ]$

d[A-G-C-G-A-A-A-G-C-A-G-$(CH_2)_n$Z]

d[A-G-C-A-A-A-A-G-C-A-G-$(CH_2)_n$Z]

d[C-T-G-C-T-T-T-$(CH_2)_n$Z]

d[C-C-T-G-C-T-T-T-T-G-C-$(CH_2)_n$Z]

d[C-C-T-G-C-T-T-T-T-G-C-$(CH_2)_n$Z]

d[C-C-T-G-C-T-T-T-C-G-C$(CH_2)_n$Z]

d[T-C-G-T-C-G-$(CH_2)_n$Z]

d[G-G-C-A-T-C-G-T-C-G-$(CH_2)_n$Z]

in which Z is an intercalating agent.

14. A pharmaceutical composition characterised in that the active principle therein is at least one drug according to any of claims 1 to 13 and a pharmaceutically acceptable excipient.

15. A pharmaceutical composition according to claim 14, characterised in that it also comprises an antibiotic, an antiparasitic agent, an antiviral agent or an antitumor agent.

**Claims for the Contracting State: AT**

1. A method of manufacturing drugs comprising oligonucleotide compounds bonded to an intercalating agent consisting of an oligonucleotide or oligodesoxynucleotide made up of a chain of natural or modified nucleotides complementary with a nucleic acid sequence, characterised in that an intercalating group is attached by a covalent bond to the aforementioned nucleic acid sequence which constitutes all or part of a sequence involved in initiation, propagation or termination of replication of a nucleic acid, and of transcription and/or translation of one or more genes.

2. A method of manufacturing drugs according to claim 1, characterised in that the oligonucleotide or oligodesoxynucleotide is complementary with a sequence for replicating or developing a virus or bacterium or parasite.

3. A method of manufacturing drugs according to claim 2, characterised in that the sequence of oligonucleotides or oligodesoxynucleotides is complementary with a region for regulation, initiation or propagation of replication.

4. A method of manufacturing drugs according to claim 1, characterised in that the sequence of oligonucleotides or oligodesoxynucleotides is complementary with a nucleic acid sequence for expressing a gene which codes for a factor giving resistance to antibiotics or antiviral agents or antiparasitic agents.

5. A method of manufacturing drugs according to claim 2 or 3, characterised in that the virus is the influenza virus or the herpes virus.

6. A method of manufacturing drugs according to claim 1, characterised in that the sequence of oligonucleotides or oligodesoxynucleotides is complementary with a nucleic acid sequence for expressing an oncogenic gene.

7. A method of manufacturing drugs according to any of claims 1 to 6 and having the formula:

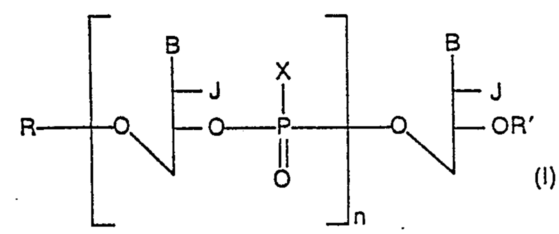

(I)

in which:

the radicals B can be identical or different and each denote a natural or modified nucleic acid base;

the radicals X, which can be identical or different, each represent an oxoanion $O^{\ominus}$ or a thioanion $S^{\ominus}$ or an alkyl group or an alkoxy, aryloxy, alkoxy or alkyl group substituted by a nitrogen-containing heterocycle, or an aminoalkyl group or an aminoalkoxy group or a thioalkyl group or a -Y-Z group;

R and R', which can be identical or different, each represent a hydrogen atom or a -Y-Z group in which:

Y represents a straight-chain or branched alkylene radical -alk- or a radical

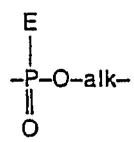

in which E can have the same meanings as X except Y-Z; or a radical -Y''-O-Y' where Y'' and Y' can have the meanings given for Y,

Z is a radical corresponding to an intercalating agent; only one out of X, R and R' is a Y-Z group;

J represents a hydrogen atom or a hydroxy group, and

n is an integer including O.

8. A method of manufacturing drugs according to claim 7, characterised in that the intercalating agent Z is chosen from among polycyclic compounds having a plane configuration.

9. A method of manufacturing drugs according to claim 8, characterised in that Z is chosen from among acridine, furocoumarin or ellpticine.

10. A method of manufacturing drugs according to any of claims 1–9, characterised in that the radical B is a radical corresponding to thymine, adenine, 2-aminoadenine and derivatives thereof substituted e.g. on the $N^6$ by an aminoalkylene group or by an azidophenylalkylene group, cytosine, guanine or guanine substituted on the $O^6$ e.g. by an (ω-alkylene)-9-acridine group, or 8-(ω-aminoalkyl)-aminoadenine and derivatives thereof substituted on the $NH_2$ at ω by an acridine group; uracil, 5-bromo-uracil, 8-azidoadenine or a photoactivatable derivative of the bases.

11. A method of manufacturing drugs according to any one of claims 1–10, characterised in that the radical Z is chosen from among:

Z'

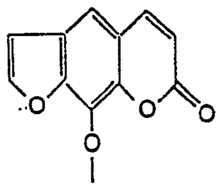

Z''

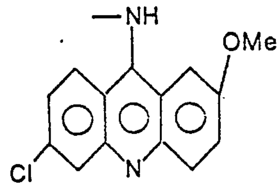

Z'''

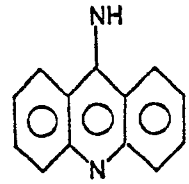

12. A method of manufacturing drugs according to any one of claims 1 to 11, characterised in that B is chosen from among T, G, A, C and U; X is chosen from among $O^-$, methyl, ethyl, propyl, methoxy, ethoxy and propoxy, and J = H.

13. A method of manufacturing drugs according to claim 1, characterised in that the compounds are chosen from among:

$(T-)_n(CH_2)_nZ$

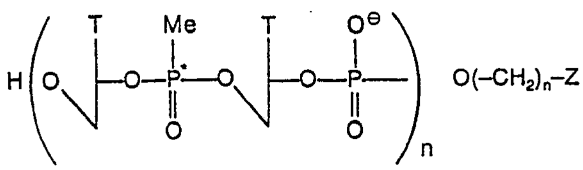

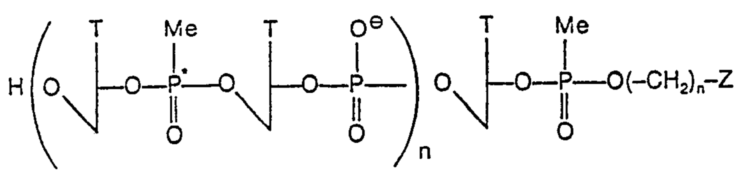

$d[T-G-A-(CH_2)_nZ]$

$d[C-C-C-T-G-A-(CH_2)_nZ]$

$d[T-A-A-C-C-C-T-G-A-(CH_2)_nZ]$

$d[A-A-A-G-C-A-G-(CH_2)_nZ]$

$d[A-G-C-G-A-A-A-G-C-A-G-(CH_2)_nZ]$

$d[A-G-C-A-A-A-A-G-C-A-G-(CH_2)_nZ]$

$d[C-T-G-C-T-T-T-(CH_2)_nZ]$

$d[C-C-T-G-C-T-T-T-T-G-C-(CH_2)_nZ]$

$d[C-C-T-G-C-T-T-T-T-G-C-(CH_2)_nZ]$

$d[C–C–T–G–C–T–T–T–C–G–C(CH_2)_nZ]$

$d[T–C–G–T–C–G–(CH_2)_nZ]$

$d[G–G–C–A–T–C–G–T–C–G–(CH_2)_nZ]$

in which Z is an intercalating agent.

14. A method of preparing a pharmaceutical composition, characterised in that the active principle therein is at least one drug according to any of claims 1 to 13 and a pharmaceutically acceptable excipient.

15. A method of preparing a pharmaceutical composition according to claim 14, characterised in that it also comprises an antibiotic, an antiparasitic agent, an antiviral agent or an antitumor agent.

**Patentansprüche für die Vertragsstaaten: BE, CH, DE, FR, GB, IT, LI, LU, NL, SE**

1. Oligonucleotidverbindungen als Arzneimittel, gebunden an ein interkalatierendes Mittel, gebildet durch ein Oligonucleotid oder ein Oligodesoxynucleotid, zusammengesetzt aus einer Verknüpfung von natürlichen oder modifizierten Nucleotiden, komplementär einer Sequenz einer Nucleinsäure, auf der sich über eine kovalente Bindung fixiert eine Interkalationsgruppe befindet, wobei die Sequenz der Nucleinsäure sich ganz oder teilweise aus einer Sequenz zusammensetzt, die die Initierung, die Propagation oder die Terminierung der Replikation einer Nucleinsäure, der Transcription und/oder der Traduktion eines oder mehrerer Gene impliziert.

2. Verbindungen nach Anspruch 1 als Arneimittel, dadurch gekennzeichnet, daß das Oligonucleotid oder daś Oligodesoxynucleotid komplementär einer Sequenz ist, die die Replikation und die Entwicklung eines Virus, eines Bakteriums oder eines Parasiten sicherstellt.

3. Verbindungen nach Anspruch 2 als Arzneimittel, dadurch gekennzeichnet, daß die Sequenz der Oligonucleotide oder der Oligodesoxynucleotide komplementär einer Region der Regulation, Initiation oder der Propagation der Replikation ist.

4. Verbindungen nach Anspruch 1 als Arzneimittel, dadurch gekennzeichnet, daß die Sequenz der Oligonucleotide oder der Oligodesoxynucleotide komplementär einer Sequenz einer Nukleinsäure ist, die die Expression eines Gens sicherstellt, das für einen Resistenzfaktor gegenüber Antibiotika, antiviralen Mitteln oder Antiparasitenmitteln kodiert.

5. Verbindungen nach einem der Ansprüche 2 und 3 als Arzneimittel, dadurch gekennzeichnet, daß der Virus der Grippevirus oder der Herpes-Virus ist.

6. Verbindungen nach Anspruch 1 als Arzneimittel, dadurch gekennzeichnet, daß die Sequenz der Oligonucleotide oder der Oligodesoxynucleotide einer Sequenz einer Nukleinsäure komplementär ist, die die Expression eines onkogenen Gens sicherstellt.

7. Verbindungen nach einem der Ansprüche 1 bis 6 als Arzneimittel, mit der Formel:

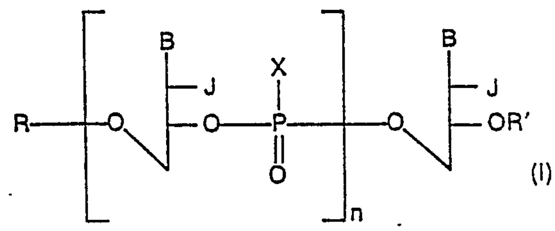

(I)

worin:

die Reste B gleich oder verschieden sein können und jeweils eine Base einer natürlichen oder modifizierten Nucleinsäure darstellen;

die Reste X, die gleich oder verschieden sein können, jeweils ein Oxoanion $O^\ominus$, ein Thioanion $S^\ominus$, eine Alkylgruppe, eine Alkoxygruppe, Aryloxy, Alkoxy oder Alkyl, substituiert durch einen Stickstoffheterozyklus, eine Aminoalkylgruppe, eine Aminoalkoxygruppe, eine Thioalkylgruppe oder eine Gruppe –Y–Z darstellen;

R und R′, die gleich oder verschieden sein können, jeweils ein Wasserstoffatom oder eine Gruppe –Y–Z bedeuten, worin:

Y ein Alkoylrest mit geradkettigen oder verzweigtem Alkylrest, oder ein Rest

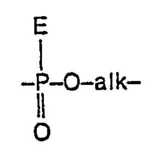

darstellt, worin E die gleichen Bedeutungen wie X haben kann mit Ausnahme Y–Z; oder bevorzugt ein Rest –Y″–O–Y′ worin Y″ und Y′ die gleichen Bedeutungen wie für Y gegeben haben können; und

Z ein Rest ist, der einem Interkalationsmittel entspricht, wobei nur einer der Reste X, R und R′ eine Gruppe Y–Z ist;

J ein Wasserstoffatom oder eine Hydroxygruppe darstellt;

n eine ganze Zahl Y eingeschlossen O ist.

8. Verbindungen nach Anspruch 7 als Arzneimittel, dadurch gekennzeichnet, daß das Interkalationsmittel Z aus den polycyclischen Verbindungen ausgewählt wird, die eine ebene Konfiguration haben.

9. Verbindungen nach Anspruch 8 als Arzneimittel, dadurch gekennzeichnet, daß Z ausgewählt wird unter Acridin, Furocoumarin oder Ellipticin.

10. Verbindungen nach einem der Ansprüche 1 bis 9 als Arzneimittel, dadurch gekennzeichnet, daß der Rest B ein Rest ist, der Thymin, Adenin, 2-Aminoadenin und dessen Derivaten, substituiert, z.B. am $N^6$, durch eine Aminoalkylengruppe oder durch eine Azidophenylalkylengruppe, Cytosin, Guanin oder Guanin substituiert an dem $O^6$, z.B. durch eine Gruppe (ω-Alkylen)-9-acridin, 8-(ω-Aminoalkyl)-amino-adenin und dessen Derivaten, substituiert an dem $NH_2$ in der ω-Position durch eine Acridingruppe; Uracil, 5-Bromuracil, 8-Azidoadenin oder einem photoaktivierbaren Derivat der Basen entspricht.

11. Verbindungen nach einem der Ansprüche 1 bis 10 als Arzneimittel, dadurch gekennzeichnet, daß der Rest Z ausgewählt wird unter:

Z'

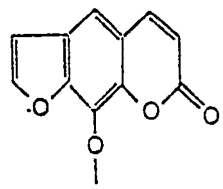

Z''

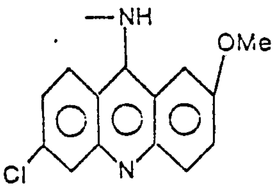

Z'''

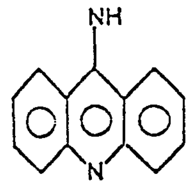

12. Verbindungen nach einem der Ansprüche 1 bis 11 als Arzneimittel, dadurch gekennzeichnet, daß B ausgewählt wird unter T, G, A, C und U; X ausgewählt wird unter $O^-$, Methyl, Ethyl, Propyl, Methoxy, Ethoxy, Propoxy und J = H.

13. Verbindungen nach Anspruch 1 als Arzneimittel, dadurch gekennzeichnet, daß die Verbindungen ausgewählt werden unter:

$(T-)_n(CH_2)_nZ$

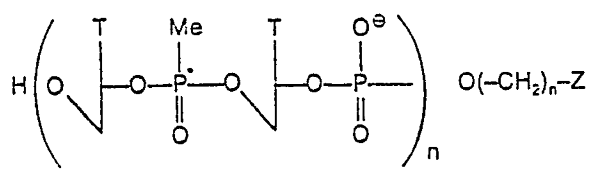

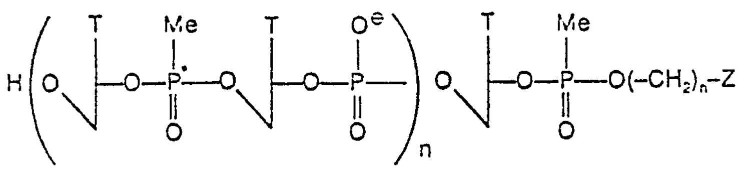

$d[T-G-A-(CH_2)_nZ]$

$d[C-C-C-T-G-A-(CH_2)_nZ]$

$d[T-A-A-C-C-C-T-G-A-(CH_2)_nZ]$

$d[A-A-A-G-C-A-G-(CH_2)_nZ]$

$d[A-G-C-G-A-A-A-G-C-A-G-(CH_2)_nZ]$

$d[A-G-C-A-A-A-A-G-C-A-G-(CH_2)_nZ]$

$d[C-T-G-C-T-T-T-(CH_2)_nZ]$

$d[C-C-T-G-C-T-T-T-T-G-C-(CH_2)_nZ]$

$d[C-C-T-G-C-T-T-T-T-G-C-(CH_2)_nZ]$

$d[C-C-T-G-C-T-T-T-C-G-C(CH_2)_nZ]$

$d[T-C-G-T-C-G-(CH_2)_nZ]$

$d[G-G-C-A-T-C-G-T-C-G-(CH_2)_nZ]$ worin Z ein Interkalationsmittel ist.

14. Pharmazeutische Zusammensetzung, dadurch gekennzeichnet, daß sie als aktiven Inhaltsstoff wenigstens ein Arzneimittel nach einem der Ansprüche 1 bis 13 und ein pharmazeutisch annehmbares Trägermaterial enthält.

15. Pharmazeutische Zusammensetzung nach Anspruch 14, dadurch gekennzeichnet, daß sie darüberhinaus ein Antibiotikum, ein Antiparasitenmittel, ein Antivirusmittel oder ein Antitumormittel enthält.

**Patentansprüche für den Vertragsstaat: AT**

1. Verfahren zur Herstellung von Arzneimitteln, bestehend aus Oligonucleotidverbindungen, gebunden an ein interkalatierendes Mittel, gebildet durch ein Oligonucleotid oder ein Oligodesoxynucleotid, zusammengesetzt aus einer Verknüpfung von natürlichen oder modifizierten Nucleotiden, komplementär einer Sequenz einer Nucleinsäure, auf der sich über eine kovalente Bindung fixiert eine Interkalationsgruppe befindet, wobei die Sequenz der Nucleinsäure sich ganz oder teilweise aus einer Sequenz zusammensetzt, die die Initierung, die Propagation oder die Terminierung der Replikation einer Nucleinsäure, der Transcription und/oder der Traduktion eines oder mehrerer Gene impliziert.

2. Verfahren zur Herstellung von Arzneimitteln nach Anspruch 1, dadurch gekennzeichnet, daß das Oligonucleotid oder das Oligodesoxynucleotid komplementär einer Sequenz ist, die die Replikation und die Entwicklung eines Virus, eines Bakteriums oder eines Parasiten sicherstellt.

3. Verfahren zur Herstellung von Arzneimitteln nach Anspruch 2, dadurch gekennzeichnet, daß die Sequenz der Oligonucleotide oder der Oligodesoxynucleotide komplementär einer Region der Regulation, Initiation oder der Propagation der Replikation ist.

4. Verfahren zur Herstellung von Arzneimitteln nach Anspruch 1, dadurch gekennzeichnet, daß die Sequenz der Oligonucleotide oder der Oligodesoxynucleotide komplementär einer Sequenz einer Nukleinsäure ist, die die Expression eines Gens sicherstellt, das für einen Resistenzfaktor gegenüber Antibiotika, antiviralen Mitteln oder Antiparasitenmitteln kodiert.

5. Verfahren zur Herstellung von Arzneimitteln nach einem der Ansprüche 2 oder 3, dadurch gekennzeichnet, daß der Virus der Grippenvirus oder der Herpes-Virus ist.

6. Verfahren zur Herstellung von Arzneimitteln nach Anspruch 1, dadurch gekennzeichnet, daß die Sequenz der Oligonucleotide oder der Oligodesoxynucleotide einer Sequenz einer Nukleinsäure komplementär ist, die die Expression eines onkogenen Gens sicherstellt.

7. Verfahren zur Herstellung von Arzneimitteln nach einem der Ansprüche 1 bis 6 mit der Formel:

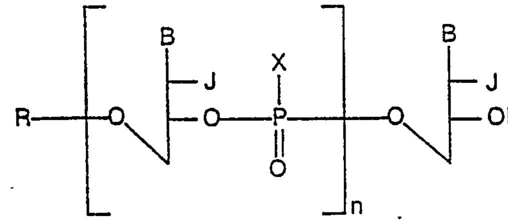

(I)

worin:

die Reste B gleich oder verschieden sein können und jeweils eine Base einer natürlichen oder modifizierten Nucleinsäure darstellen:

die Reste X, die gleich oder verschieden sein können, jeweils ein Oxoanion $O^{\ominus}$, ein Thioanion $S^{\ominus}$, eine Alkylgruppe, eine Alkoxygruppe, Aryloxy, Alkoxy oder Alkyl, substituiert durch einen Stickstoffheterozyklus, eine Aminoalkylgruppe, eine Aminoalkoxygruppe, eine Thioalkylgruppe oder eine Gruppe –Y–Z darstellen;

R und R', die gleich oder verschieden sein können, jeweils ein Wasserstoffatom oder eine Gruppe –Y–Z bedeuten, worin;

Y ein Alkoylrest mit gradkettigen oder verzweigtem Alkylrest, oder ein Rest

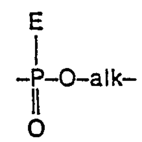

darstellt, worin E die gleichen Bedeutungen wie X haben kann mit Ausnahme Y–Z; oder bevorzugt ein Rest –Y''–O–Y' worin Y'' und Y' die gleichen Bedeutungen wie für Y gegeben haben können; und

Z ein Rest ist, der einem Interkalationsmittel entspricht, wobei nur einer der Reste X, R und R' eine Gruppe Y–Z ist;

J ein Wasserstoffatom oder eine Hydroxygruppe darstellt;

n eine ganze Zahl Y eingeschlossen 0 ist.

8. Verfahren zur Herstellung von Arzneimitteln nach Anspruch 7, dadurch gekennzeichnet, daß das Interkalationsmittel Z aus den polycyclischen Verbindungen ausgewählt wird, die eine ebene Konfiguration haben.

9. Verfahren zur Herstellung von Arzneimitteln nach Anspruch 8, dadurch gekennzeichnet, daß Z ausgewählt wird unter Acridin, Furocoumarin oder Ellipticin.

10. Verfahren zur Herstellung von Arzneimitteln nach einem der Ansprüche 1 bis 9, dadurch gekennzeichnet, daß der Rest B ein Rest ist, der Thymin, Adenin, 2-Aminoadenin und dessen Derivaten, substituiert, z.B. am $N^6$, durch eine Aminoalkylengruppe oder durch eine Azidophenylalkylengruppe, Cytosin, Guanin oder Guanin substituiert an dem $O^6$, z.B. durch eine Gruppe (ω-Alkylen)-9-acridin, 8-(ω-Aminoalkyl)-amino-adenin und dessen Derivaten, substituiert an dem $NH_2$ in der ω-Position durch eine Acridingruppe; Uracil, 5-Bromuracil, 8-Azidoadenin oder einem photoaktivierbaren Derivat der Basen entspricht.

11. Verfahren zur Herstellung von Arzneimitteln nach einem der Ansprüche 1 bis 10, dadurch gekennzeichnet, daß der Rest Z ausgewählt wird unter:

Z'

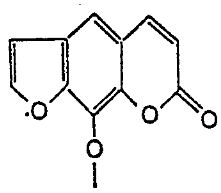

Z''

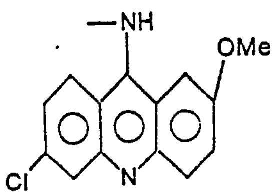

Z'''

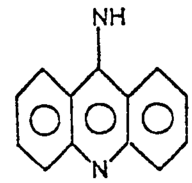

12. Verfahren zur Herstellung von Arzneimitteln nach einem der Ansprüche 1 bis 11, dadurch gekennzeichnet, daß B ausgewählt wird unter T, G, A, C und U; X ausgewählt wird unter $O^-$, Methyl, Ethyl, Propyl, Methoxy, Ethoxy, Propoxy und J = H.

13. Verfahren zur Herstellung von Arzneimitteln nach Anspruch 1, dadurch gekennzeichnet, daß die Verbindungen ausgewählt werden unter:

$(T-)_n(CH_2)_nZ$

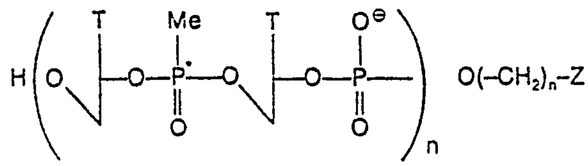

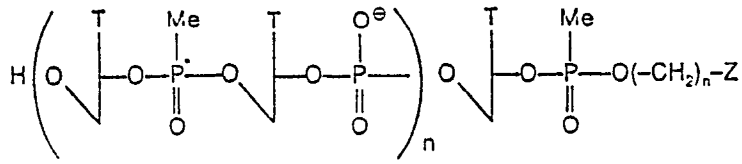

$d[T-G-A-(CH_2)_nZ]$

$d[C-C-C-T-G-A-(CH_2)_nZ]$

$d[T-A-A-C-C-C-T-G-A-(CH_2)_nZ]$

$d[A-A-A-G-C-A-G-(CH_2)_nZ]$

$d[A-G-C-G-A-A-A-G-C-A-G-(CH_2)_nZ]$

$d[A-G-C-A-A-A-A-G-C-A-G-(CH_2)_nZ]$

$d[C-T-G-C-T-T-T-(CH_2)_nZ]$

$d[C-C-T-G-C-T-T-T-T-G-C-(CH_2)_nZ]$

$d[C-C-T-G-C-T-T-T-T-G-C-(CH_2)_nZ]$

$d[C-C-T-G-C-T-T-T-C-G-C(CH_2)_nZ]$

$d[T-C-G-T-C-G-(CH_2)_nZ]$

$d[G-G-C-A-T-C-G-T-C-G-(CH_2)_nZ]$

worin Z ein Interkalationsmittel ist.

14. Verfahren zur Herstellung einer pharmazeutischen Zusammensetzung, dadurch gekennzeichnet, daß man als aktiven Inhaltsstoff wenigstens ein Arzneimittel, hergestellt nach einem der Ansprüche 1 bis 13, und ein pharmazeutisch annehmbares Trägermaterial zusammenfügt.

15. Verfahren zur Herstellung einer pharmazeutischen Zusammensetzung nach Anspruch 14, dadurch gekennzeichnet, daß man dieser darüberhinaus ein Antibiotikum, ein Antiparasitenmittel, ein Antivirusmittel oder ein Antitumormittel hinzufügt.